# EUROPEAN PATENT APPLICATION

(11) **EP 4 112 724 A1**
(43) Date of publication of application: **04.01.2023**
(21) Application number: 21763930.1
(22) Date of filing: 04.03.2021
(51) Int. Cl.: C12N 7/00, C12N 15/869, A61K 35/763, A61K 48/00, A61K 38/20, A61K 38/19, A61K 39/395, A61P 35/00, A61P 7/00, A61P 7/02, A61P 25/00, A61P 25/04, A61P 37/02, A61P 25/16, C12R 1/93

(54) **HERPES SIMPLEX VIRUS AND USE THEREOF**

(30) Priority: 05.03.2020 WO PCT/CN2020/077971
(71) Applicant: Beijing Wellgene Biotech Co., Ltd., Beijing 100085 (CN)
(72) Inventor: ZHAO, Jingshu, Beijing 100085 (CN); LI, Xiaopeng, Beijing 100085 (CN); TIAN, Chao, Beijing 100085 (CN); ZHOU, Hua, Beijing 100085 (CN); WANG, Tiantian, Beijing 100085 (CN)
(74) Representative: karo IP
(86) International application number: PCT/CN2021/079114
(87) International publication number: WO 2021/175293

(57) **Abstract**

Provided are a new herpes simplex virus type I, a genetically modified herpes simplex virus, a composition containing the virus, a host cell and a cell culture, and the use of the virus in the treatment of diseases.

## Description

### FIELD OF THE APPLICATION

The present application relates to a novel herpes simplex virus, modification of the virus, and a genetically modified herpes simplex virus and the use thereof.

### BACKGROUND OF THE APPLICATION

Herpes Simplex Virus (HSV) belongs to herpesviridae family and is a double-stranded DNA virus with an envelope, including two subtypes, type I (HSV-1) and type II (HSV-2).

Herpes simplex virus consists of four parts: core, capsid, tegument protein and envelope. The core of the virus consists of a thick double-stranded DNA that is twisted into fibrillar spools. The capsid outside and surrounding the DNA is icosahedral in structure with a diameter of 100-110 nm and consists of 162 capsomeres. The capsid is covered by irregularly arranged, amorphous tegument proteins. The outermost layer of the virus is a lipid double-layered envelope with short protrusions, and the diameter of the virus wrapped with the envelope is 150-200 nm. The viral envelope consists of 11 glycoproteins and at least two non-glycosylated proteins, including viral glycoprotein B (gB), viral glycoprotein C (gC), viral glycoprotein D (gD), viral glycoprotein G (gG) and viral glycoprotein M (gM), etc. The stereostructure of the viral envelope glycoprotein is related to the ability of the virus to infect cells, which determines whether the virus can enter the host cell and the amount of virus that enters the host cell.

At present, HSV-1 virus has been used for gene therapy of major diseases such as tumor, neurodegenerative diseases, genetic diseases and immune system diseases, etc. Compared with other gene therapy virus vectors, HSV-1 virus has many advantages.

First, HSV-1 has a large genome and can carry larger or multiple exogenous genes [Roizman B.The function of herpes simplex virus genes: a primer for genetic engineering of novel vectors.[J].Proceedings of the National Academy of Sciences, 1996,93(21):11307-11312]. The HSV-1 genome is 152 kb long, whereas the adenovirus genome is only 35 kb long. Among the more than 80 known genes in the HSV-1 genome, about half are nonessential in *in vitro* culture and can be replaced by multiple exogenous therapeutic genes. Its maximum exogenous gene insertion can reach 30-40 kb. This is particularly important for the treatment of many diseases, especially those associated with multiple genes.

HSV-1 does not integrate with cellular DNA, replication is controlled, and safety is high. HSV-1 produces little life-threatening disease in immunocompetent adults [Shen Y, Nemunaitis J. Herpes simplex virus 1 (HSV-1) for cancer treatment[J]. Cancer Gene Therapy, 2006, 13(11):975-992]. Many non-essential genes of HSV-1 are associated with its neurotoxicity. Removal of certain nonessential genes in HSV-1, such as the ICP34.5 gene, allows HSV-1 to replicate selectively only in tumor cells, leaving normal tissue cells unaffected by it [Chou J,Kern E,Whitley R,et al. Mapping of herpes simplex virus-1 neurovirulence to gamma 34.5, a gene nonessential for growth in culture[J]. Science, 1990, 250(4985):1262-1266].

Based on these many advantages above, HSV-1 has become one of the most widely used virus vectors for gene therapy today. In gene therapy research, HSV-1 is usually transformed into oncolytic herpes simplex virus type 1 (oHSV-1) by means of genetic modification, and the main methods include knocking out certain genes and inserting tumor therapy-related genes, etc. A variety of oHSV-1 has been studied in preclinical and clinical trials, and a large number of results have shown its good safety and efficacy.

The general strategy for the construction of gene-deficient oHSV-1 is to mutate or delete single or multiple genes such as ICP34.5, ICP6, ICP0, TK and UNG genes of the virus to achieve its tumor-specific killing effect. Talimogene laherparepvec, HSV1716, NV1020, G207, G47Δ, etc. which have been marketed and entered clinical trials, have the ICP34.5 gene knocked out in HSV-1. The oncolytic virus HSV1716 has been knocked out only the double copies of ICP34.5 gene, showed tumor regression and no significant toxicity or side effects in clinical trials for refractory or recurrent high-grade glioma. In G207, the ICP6 gene is mutated while the double copies of ICP34.5 gene are knocked out. In a clinical trial for the treatment of recurrent and progressive malignant glioma, it was shown to have a good safety profile with no serious adverse effects after intratumoral injection of 10⁹ pfu of G207 [Markert JM, Liechty PG, Wang W, et al. Phase Ib trial of mutant herpes simplex virus G207 inoculated pre- and post-tumor resection for recurrent GBM[J]. Molecular Therapy, 2009, 17(1):199-207]. Talimogene laherparepvec is the first oncolytic virus approved for marketing in the United States, which has the ICP34.5 and ICP47 genes of HSV-1 knocked out, and phase III clinical trial data show a 26.4% objective remission rate in the treatment of malignant melanoma, which has a relatively significant therapeutic effect and good safety profile [Andtbacka R H I, Kaufman H L , Collichio F , et al. Talimogene Laherparepvec Improves Durable Response Rate in Patients With Advanced Melanoma[J]. Journal of Clinical Oncology, 2015, 33(25):2780-2788]. These clinical data suggest that HSV-1 has a good safety profile and great potential for gene therapy of tumors.

Herpes simplex virus type I (HSV-1) is considered to be a very promising virus for gene therapy because of its broad host cell range, high safety profile and neurophilic nature. For example, herpes simplex virus type 1 can be genetically modified to become a non-replicating virus which introduces exogenous genes into the subject to treat related diseases. For example, in pain treatment, by injection of HSV-1 vectors carrying ENK, GABA or endorphin genes, chronic inflammatory and bone cancer-induced pain as well as neuropathic pain can be reduced, while HSV-1 vectors carrying antiinflammatory factors such as IL-4, IL-10 and IL-13 can also relieve acute and chronic pain [Goss J R, Krisky D M, Wechuck J B, et al. Herpes simplex virus-based nerve targeting gene therapy in pain management[J]. Journal of Pain Research, 2014: 71-79].

Most of the herpes simplex viruses currently in use for gene delivery or therapy are laboratory strains that have been passaged in tissue culture cells for many years. Although these viruses can enter cells for gene delivery, there is room for improvement in the properties required for disease treatment such as the ability to transfect and express exogenous genes.

### SUMMARY OF THE APPLICATION

The present application, on the one hand, aims to overcome the problems of the prior art by providing a new HSV isolate that has a significantly improved ability to infect human cells *in vivo* and a significantly enhanced ability to replicate/cleave intracellularly relative to the continuously passaged standard model strain HSV-1 virus strain 17 (referred to as virus strain 17 or strain 17) that has been used previously, and is suitable as an oncolytic virus or as a vector for destroying tumor cells through oncolysis.

Compared to the standard model strain HSV-1 virus strain 17, the new HSV-1 isolate (referred to as virus strain HL-1, strain HL-1 or HL-1) provided in the present application has enhanced replication in human tumor cell lines. In the absence of ICP34.5 and/or ICP47, virus strain HL-1 showed significantly enhanced replication ability within human tumor cell lines, and killing ability to tumor cells, compared to an HSV-1 virus strain 17 in which ICP34.5 and/or ICP47 were also absent.

The modified HL-1 virus of the present application was subsequently used to deliver genes with antitumor activity, which was shown to further enhance antitumor immune response activity and oncolysis.

On the other hand, the present application also provides a non-replicating recombinant herpes virus. The non-replicating recombinant herpes virus is derived from a herpes simplex virus with a CCTCC deposit number of V201810, wherein one or more of the immediate early genes are knocked out, or the expression of which is blocked or reduced by gene mutation. The non-replicating recombinant herpes virus of the present application has significantly enhanced transfection ability and ability to express exogenous genes compared to herpesviruses of the prior art. Therefore, the non-replicating recombinant herpesvirus of the present application also has potential developmental value in introducing exogenous genes into patients for the treatment of, for example, nervous system diseases, hematological system diseases, immune system diseases, and other diseases.

Specifically, the present application relates, in one aspect, to each of the following:
1. A herpes simplex virus type 1 (HSV-1) strain, having one or more properties selected from the group consisting of the following:
   (a) compared to a reference laboratory strain, having an enhanced ability to infect tumor cells;
   (b) compared to a reference laboratory strain, having an enhanced ability to replicate within tumor cells;
   (c) compared to a reference laboratory strain, having an enhanced ability to kill tumor cells;
   (d) after modification, the herpes simplex virus has an enhanced ability to infect tumor cells compared to a reference laboratory strain with equivalent modification;
   (e) after modification, the herpes simplex virus has an enhanced ability to replicate within tumor cells compared to a reference laboratory strain with equivalent modification; and
   (f) after modification, the herpes simplex virus has an enhanced ability to kill tumor cells compared to a reference laboratory strain with equivalent modification;
   wherein the reference laboratory strain is a standard model strain HSV-1 virus strain 17.
2. The herpes simplex virus type 1 strain of item 1, which is a virus strain with a CCTCC deposit number of V201810 (CCTCC: V201810).
3. the herpes simplex virus type 1 strain of item 1, which is an attenuated strain of the virus strain with a CCTCC deposit number of V201810 (CCTCC: V201810) or a progeny thereof.

In some embodiments, the present application relates to genetically engineered constructs of the virus strain with a CCTCC deposit number of V201810 (CCTCC: V201810).
4. A herpes simplex virus derived from the virus strain with a CCTCC deposit number of V201810 (CCTCC: V201810), wherein the herpes simplex virus has one or more genes selected from the group consisting of ICP34.5 gene, ICP6 gene, ICP0 gene, ICP47 gene, US3 gene, UL56 gene, functional VP16 gene, VHS gene, UNG gene, glycoprotein H gene, and thymidine kinase gene disrupted or knocked out.
5. The herpes simplex virus of item 4, wherein the ICP34.5 gene and/or the ICP47 gene are disrupted or knocked out.
6. A composition comprising the herpes simplex virus of any one of items 1-5.
7. The composition of item 6, which is a virus culture.
8. The host cell infested with the herpes simplex virus of any one of items 1-5.
9. A composition comprising the host cell of item 8.
10. The composition of item 9, which is a cell culture.
11. A method for preparing herpes simplex virus type 1 (HSV-1), comprising
   1) clinically collecting a herpes simplex virus strain;
   2) screening a virus strain having one or more properties selected from the group consisting of:
      (a) compared to a reference laboratory strain, having an enhanced ability to infect tumor cells;
      (b) compared to a reference laboratory strain, having an enhanced ability to replicate within tumor cells;
      (c) compared to a reference laboratory strain, having an enhanced ability to kill tumor cells;
      (d) after modification, the herpes simplex virus has an enhanced ability to infect tumor cells compared to a reference laboratory strain with equivalent modification;
      (e) after modification, the herpes simplex virus has an enhanced ability to replicate within tumor cells compared to a reference laboratory strain with equivalent modification; and
      (f) after modification, the herpes simplex virus has an enhanced ability to kill tumor cells compared to a reference laboratory strain with equivalent modification;
      wherein the reference laboratory strain is a standard model strain HSV-1 virus strain 17.
12. The method for preparing herpes simplex virus type 1 (HSV-1) of item 11, comprising:
   1) clinically collecting a herpes simplex virus strain;
   2) screening a virus strain having the following properties:
      (a) compared to the reference laboratory strain, having an enhanced ability to infect tumor cells;
      (b) compared to the reference laboratory strain, having an enhanced ability to replicate within tumor cells; and
      (c) compared to the reference laboratory strain, having an enhanced ability to kill tumor cells.
13. Herpes simplex virus type 1 (HSV-1) obtained by the preparation method of item 11 or 12.

On the one hand, the present application also relates to each of the following:
1. A genetically modified herpes simplex virus, which is derived from a herpes simplex virus with a CCTCC deposit number of V201810, wherein one or more genes selected from the group consisting of: ICP34.5 gene, ICP6 gene, ICP0 gene, ICP47 gene, US3 gene, UL56 gene, functional VP16 gene, VHS gene, UNG gene, glycoprotein H gene and thymidine kinase gene are disrupted or knocked out.
2. The genetically modified herpes simplex virus of item 1, wherein the ICP34.5 gene and/or the ICP47 gene is disrupted or knocked out.
3. The genetically modified herpes simplex virus of item 1 or 2, wherein one or more exogenous genes are introduced into the virus.
4. The genetically modified herpes simplex virus of item 3, wherein the one or more exogenous genes are inserted into one or more deletion sites of a gene selected from the group consisting of: ICP34.5 gene, ICP6 gene, ICP0 gene, ICP47 gene, US3 gene, UL56 gene, functional VP16 gene, VHS gene, UNG gene, glycoprotein H gene, and thymidine kinase gene.
5. The genetically modified herpes simplex virus of item 4, wherein the exogenous genes are one or more selected from the group consisting of: genes encoding cytokines promoting an immune response, genes encoding tumor antigens, genes encoding monoclonal antibodies having prophylactic and/or therapeutic effects against tumors, genes encoding prodrug-converting enzymes, tumor suppressor genes, antisense RNAs, or small RNAs.
6. The herpes simplex virus of item 5, wherein the cytokines are one or more selected from the group consisting of: GM-CSF, G-CSF, M-CSF, IL-1, IL-2, IL-3, IL-4, IL-5, IL-6, IL7, IL-8, IL-10, IL-12, IL-13, IL-15, IL-18, IL-21, IL-23 , IFN-α, IFN-γ, TGF-β, and TNF-α.
7. The herpes simplex virus of item 5, wherein the monoclonal antibodies having prophylactic and/or therapeutic effects against tumors are one or more selected from the group consisting of: anti-PD-1 monoclonal antibodies, anti-PD-L1 monoclonal antibodies, anti-PD-L2 monoclonal antibodies, anti-CTLA-4 monoclonal antibodies, anti-CD80 monoclonal antibodies, anti-CD28 monoclonal antibodies, anti-CD137 monoclonal antibodies, anti-CD137L monoclonal antibodies, anti-OX40 monoclonal antibodies, anti-OX40L monoclonal antibodies, anti-CD27 monoclonal antibodies, anti-CD70 monoclonal antibodies, anti-CD40 monoclonal antibodies, anti-CD40L monoclonal antibodies, anti-LAG-3 monoclonal antibodies, and anti-TIM-3 monoclonal antibodies.
8. The herpes simplex virus of item 5, wherein the tumor antigens are one or more selected from the group consisting of: PSA, MUC1, MAGE-1, MAGE-2, MAGE-3, MAGE-12, BAGE, GAGE, and LAGE.
9. The herpes simplex virus of item 5, wherein the prodrug-converting enzyme is a cytidine deaminase or a herpes simplex virus thymidine kinase.
10. The herpes simplex virus of item 5, wherein the tumor suppressor gene is P53 or PTEN.
11. The herpes simplex virus of item 5, wherein the antisense RNAs or small RNAs are RNA fragments that block or downregulate tumor-overexpressed proto-oncogenes, or metabolic genes.
12. The genetically modified herpes simplex virus of item 5, wherein the exogenous genes encode at least one, two, or three selected from the group consisting of cytokine polypeptides stimulating immune response and antibody polypeptides promoting immune response.
13. The genetically modified herpes simplex virus of item 12, wherein the cytokine polypeptides stimulating an immune response are one or more selected from the group consisting of: granulocyte macrophage colony-stimulating factor (GMCSF), IL-2, IL12, IFN-γ, and TNF-α.
14. The genetically modified herpes simplex virus of item 12, wherein the antibody polypeptides promoting an immune response are immune checkpoint antibody polypeptides.
15. The genetically modified herpes simplex virus of item 14, wherein the immune checkpoint antibody is an anti-PD-1 antibody or an anti-PD-L1 antibody.
16. The genetically modified herpes simplex virus of any one of items 1-4, further comprising disrupting or knocking out one or more immediate early genes.
17. The genetically modified herpes simplex virus of item 16, wherein the one or more immediate early genes are one, two, three or four selected from the group consisting of genes encoding ICP0, ICP4, ICP22 and ICP27.
18. A composition comprising the genetically modified herpes simplex virus of any one of items 1-17.
19. The composition of item 18, wherein the genetically modified herpes simplex virus has one, two, three or four exogenous genes inserted at the same site or at different sites.

On the one hand, the present application further relates to each of the following:
1. A herpes simplex virus vector, which is derived from a herpes simplex virus with a CCTCC deposit number of V201810.
2. The herpes simplex virus vector of item 1, wherein one or more genes selected from the group consisting of ICP34.5 gene, ICP6 gene, ICP0 gene, ICP47 gene, US3 gene, UL56 gene, VHS gene, UNG gene, glycoprotein H gene, and thymidine kinase gene are disrupted or knocked out.
3. Use of the herpes simplex virus vector of item 1 or 2 in the preparation of a recombinant oncolytic virus, wherein one or more exogenous genes are introduced into the recombinant oncolytic virus.
4. The use of item 3, wherein the one or more exogenous genes are inserted into one or more deletion sites of a gene selected from the group consisting of: ICP34.5 gene, ICP6 gene, ICP0 gene, ICP47 gene, US3 gene, UL56 gene, VHS gene, UNG gene, glycoprotein H gene, and thymidine kinase gene.
5. The use of item 4, wherein the exogenous genes are one or more selected from the group consisting of: genes encoding cytokines promoting an immune response, genes encoding tumor antigens, genes encoding monoclonal antibodies having prophylactic and/or therapeutic effects against tumors, genes encoding prodrug-converting enzymes, tumor suppressor genes, antisense RNAs, or small RNAs.
6. The use of item 5, wherein the cytokines are one or more selected from the group consisting of: GM-CSF, G-CSF, M-CSF, IL-1, IL-2, IL-3, IL-4, IL-5, IL-6, IL7, IL-8, IL-10, IL-12, IL-13, IL-15, IL-18, IL-21, IL-23, IFN -α, IFN-γ, TGF-β, and TNF-α.
7. The use of item 5, wherein the monoclonal antibodies having prophylactic and/or therapeutic effects against tumors are one or more selected from the group consisting of: anti-PD-1 monoclonal antibodies, anti-PD-L1 monoclonal antibodies, anti-PD-L2 monoclonal antibodies, anti-CTLA-4 monoclonal antibodies, anti-CD80 monoclonal antibodies, anti-CD28 monoclonal antibodies, anti-CD137 monoclonal antibodies, anti-CD137L monoclonal antibodies, anti-OX40 monoclonal antibodies, anti-OX40L monoclonal antibodies, anti-CD27 monoclonal antibodies, anti-CD70 monoclonal antibodies, anti-CD40 monoclonal antibodies, anti-CD40L monoclonal antibodies, anti-LAG-3 monoclonal antibodies, and anti-TIM-3 monoclonal antibodies.
8. The use of item 5, wherein the tumor antigens are tumor-derived specific antigens which are one or more selected from the group consisting of: PSA, MUC1, MAGE-1, MAGE-2, MAGE-3, MAGE-12, BAGE, GAGE, and LAGE.
9. The use of item 5, wherein the prodrug converting enzyme is a cytidine deaminase or a herpes simplex virus thymidine kinase.
10. The use of item 5, wherein the tumor suppressor gene is P53 or PTEN.
11. The use of item 5, wherein the antisense RNAs or small RNAs are RNA fragments that block or downregulate tumor-overexpressed proto-oncogenes, or metabolic genes.
12. The use of embodiment 5, wherein the cytokines that promote an immune response are granulocyte macrophage colony stimulating factor (GMCSF), IL-2, IL12, IFN-γ and/or TNF-α.
13. The use of item 5, wherein the monoclonal antibodies having prophylactic and/or therapeutic effects against tumors are immune checkpoint antibodies.
14. The use of item 13, wherein the immune checkpoint antibody is an anti-PD-1 antibody or an anti-PD-L1 antibody.
15. A method for preparing a recombinant oncolytic virus, comprising introducing one or more exogenous genes into a herpes simplex virus, wherein the herpes simplex virus is derived from a herpes simplex virus with a CCTCC deposit number of V201810.
16. The method of item 15, wherein the herpes simplex virus has one or more genes selected from the group consisting of ICP34.5 gene, ICP6 gene, ICP0 gene, ICP47 gene, US3 gene, UL56 gene, VHS gene, UNG gene, glycoprotein H gene, and thymidine kinase gene disrupted or knocked out.
17. The method of item 16, wherein the ICP34.5 gene and/or the ICP47 gene is deleted from the herpes simplex virus.
18. The method of any one of items 15-17, wherein the one or more exogenous genes are inserted into one or more deletion sites of a gene selected from group consisting of: ICP34.5 gene, ICP6 gene, ICP0 gene, ICP47 gene, US3 gene, UL56 gene, VHS gene, UNG gene, glycoprotein H gene, and thymidine kinase gene.
19. The method of item 18, wherein the exogenous genes are one or more selected from the group consisting of: genes encoding cytokines promoting an immune response, genes encoding tumor antigens, genes encoding monoclonal antibodies having prophylactic and/or therapeutic effects against tumors, genes encoding prodrug-converting enzymes, tumor suppressor genes, antisense RNAs, or small RNAs.
20. The method of item 19, wherein the cytokines are one or more selected from the group consisting of: GM-CSF, G-CSF, M-CSF, IL-1, IL-2, IL-3, IL-4, IL-5, IL-6, IL7, IL-8, IL-10, IL-12, IL-13, IL-15, IL-18, IL-21, IL-23 IFN-α, IFN-γ, TGF-β, and TNF-α.
21. The method of item 19, wherein the monoclonal antibodies having prophylactic and/or therapeutic effects against tumors are one or more selected from the group consisting of: anti-PD-1 monoclonal antibodies, anti-PD-L1 monoclonal antibodies, anti-PD-L2 monoclonal antibodies, anti-CTLA-4 monoclonal antibodies, anti-CD80 monoclonal antibodies, anti-CD28 monoclonal antibodies, anti-CD137 monoclonal antibodies, anti-CD137L monoclonal antibodies, anti-OX40 monoclonal antibodies, anti-OX40L monoclonal antibodies, anti-CD27 monoclonal antibodies, anti-CD70 monoclonal anti-CD40 monoclonal antibodies, anti-CD40L monoclonal antibodies, anti-LAG-3 monoclonal antibodies, and anti-TIM-3 monoclonal antibodies.
22. The method of item 19, wherein the tumor antigens are tumor-derived specific antigens, which are one or more selected from the group consisting of: PSA, MUC1, MAGE-1, MAGE-2, MAGE-3, MAGE-12, BAGE, GAGE, and LAGE.
23. The method of item 19, wherein the prodrug-converting enzyme is a cytidine deaminase or a herpes simplex virus thymidine kinase.
24. The method of item 19, wherein the tumor suppressor gene is P53 or PTEN.
25. The method of item 19, wherein the antisense RNAs or small RNAs are RNA fragments that block or downregulate tumor-overexpressed proto-oncogenes, or metabolic genes.
26. The method of item 18, wherein the exogenous genes encode cytokine polypeptides stimulating an immune response and/or antibody polypeptides promoting an immune response.
27. The method of item 26, wherein the cytokine polypeptides stimulating an immune response are granulocyte macrophage colony stimulating factor (GMCSF), IL-2, IL12, IFN-γ, and/or TNF-α.
28. The method of item 26, wherein the antibody polypeptides promoting an immune response are immune checkpoint antibody polypeptides.
29. The method of item 28, wherein the immune checkpoint antibody is an anti-PD-1 antibody or an anti-PD-L1 antibody.
30. The recombinant oncolytic virus prepared by the method of any one of items 15-29.
31. A composition comprising the recombinant oncolytic virus of item 30.
32. The composition of item 31, wherein the herpes simplex virus has one, two, three or four exogenous genes inserted at the same site or at different sites.
33. The composition of item 31 or 32, wherein the recombinant oncolytic virus is a mixture of two, three, four or four recombinant oncolytic viruses prepared by the method of any one of items 15-29, wherein each recombinant oncolytic virus is introduced with a different exogenous gene.
34. The composition of any one of items 31-33, which is a virus culture.

In some embodiments, the present application relates to:
1. A genetically modified herpes simplex virus, wherein one or more immediate early genes are knocked out or disrupted, or the expression of which is prevented or reduced by gene mutation, thereby losing the ability to replicate, the herpes simplex virus is derived from a virus strain with a CCTCC deposit number of V201810, an attenuated strain thereof, or a progeny thereof.
2. The herpes simplex virus of item 1, wherein the one or more immediate early genes are one, two, three or four selected from the group consisting of the genes encoding ICP0, ICP4, ICP22 and ICP27.
3. The herpes simplex virus of item 1 or 2, which is derived from the virus strain with a CCTCC deposit number of V201810.
4. The herpes simplex virus of any one of items 1-3, wherein one or more genes selected from the group consisting of ICP34.5 gene, ICP6 gene, ICP0 gene, ICP47 gene, US3 gene, UL56 gene, functional VP16 gene, VHS gene, UNG gene, glycoprotein H gene, and thymidine kinase gene are disrupted or knocked out.
5. The herpes simplex virus of item 4, wherein the ICP34.5 gene is disrupted or knocked out.
6. The herpes simplex virus of item 5, wherein the ICP47 gene is disrupted or knocked out.
7. The herpes simplex virus of any one of items 1-6, wherein one or more exogenous genes are introduced.
8. The herpes simplex virus of item 7, wherein the exogenous genes are nerve growth factor genes, neurotransmitter genes and/or neurotrophic factor genes.
9. The herpes simplex virus of item 7, wherein the exogenous genes are coagulation factor genes.
10. A composition comprising the herpes simplex virus of any one of items 1-9.
11. The composition of item 10, which is a virus culture.
12. A host cell comprising the herpes simplex virus of any one of items 1-9.
13. A composition comprising the host cell of item 12.
14. The composition of item 13, which is a cell culture.
15. Use of a herpes simplex virus with a CCTCC deposit number of V201810 or an attenuated variant thereof or a progeny thereof as a vector for introducing exogenous genes of nerve growth factors, neurotransmitters and/or neurotrophic factors into cells.
16. Use of a herpes simplex virus with a CCTCC deposit number of V201810 or an attenuated variant thereof or a progeny thereof in the preparation of a medicament for the treating nervous system disease, hematological system diseases, or immune system diseases.
17. The use of item 16, wherein the hematological system diseases are blood coagulation disorders, such as hemophilia.
18. Use of the herpes simplex virus of any one of items 1-9 in the preparation of a medicament for treating or preventing central nervous system diseases or peripheral nervous system diseases.
19. The use of item 18, wherein the central nervous system disease is stroke, paralysis or a neurodegenerative disease.
20. The use of embodiment 19, wherein the neurodegenerative disease is Parkinson's disease, Alzheimer's disease, amyotrophic lateral sclerosis, or Huntington's disease.
21. The use of item 18, wherein the peripheral nervous system diseases include motor neuron diseases, chronic pain, and peripheral nerve injury.
22. Use of a vector comprising the herpes simplex virus of any one of items 1-9 in the preparation of a medicament for the treatment or prevention of pain or nerve injury.
23. The use of item 22, wherein the treatment or prevention of pain or nerve injury comprises reducing the severity of pain or promoting nerve regeneration growth.

The present application also relates to a non-replicating herpes simplex virus vector wherein one or more immediate early genes of the herpes simplex virus are knocked out or disrupted thereby losing replication capacity, or their expression is blocked or reduced by gene mutation thereby losing replication capacity. In some embodiments, the one or more immediate early genes are one, two, three, or four selected from the genes encoding ICP0, ICP4, ICP22, ICP47, and ICP27. In some embodiments, the herpes simplex virus is derived from a herpes simplex virus with a CCTCC deposit number of V201810 or a progeny thereof. In some embodiments, one or more of ICP34.5 gene, ICP6 gene, ICP0 gene, ICP47 gene, US3 gene, UL56 gene, VP16 gene, VHS gene, UNG gene, glycoprotein H gene, and thymidine kinase gene of the herpes simplex virus is disrupted or knocked out. In some embodiments, the ICP34.5 gene of the herpes simplex virus is disrupted or knocked out. In some embodiments, the ICP47 gene of the herpes simplex virus is disrupted or knocked out. In some embodiments, the herpes simplex virus vector further comprises one or more exogenous genes introduced. In some embodiments, the exogenous genes are nerve growth factor genes, neurotransmitter genes, and/or neurotrophic factor genes. In some embodiments, the exogenous genes are coagulation factor genes.

The present application also relates to a non-replicating herpes simplex virus derived from a herpes simplex virus with a CCTCC deposit number of V201810, wherein one or more immediate early genes of the herpes simplex virus are knocked out or disrupted thereby losing replication capacity, or their expression is blocked or reduced by gene mutation thereby losing replication capacity. In some embodiments, the one or more immediate early genes are one, two, three, or four selected from the group consisting of genes encoding ICP0, ICP4, ICP22, ICP47, and ICP27. In some embodiments, one or more of the ICP34.5 gene, ICP6 gene, ICP0 gene, ICP47 gene, US3 gene, UL56 gene, VP16 gene, VHS gene, UNG gene, glycoprotein H gene, and thymidine kinase gene of the herpes simplex virus are disrupted or knocked out. In some embodiments, the ICP34.5 gene of the herpes simplex virus is disrupted or knocked out. In some embodiments, the ICP47 gene of the herpes simplex virus is disrupted or knocked out. In some embodiments, exogenous genes, such as a nerve growth factor gene, neurotransmitter genes, neurotrophic factor genes, or coagulation factor genes, are introduced into the virus.

### BRIEF DESCRIPTION OF THE DRAWINGS

Figure 1: 25000x electron micrograph of virus strain HL-1.
Figure 2: HepG2 cells transfected with different MOI (multiplicity of infection) of HL-1 and HSV (17).
Figure 3: A375 cells transfected with different MOI of HL-1 and HSV(17).
Figure 4: A549 cells transfected with different MOI of HL-1 and HSV(17).
Figure 5: MCF-7 cells transfected with different MOI of HL-1 and HSV(17).
Figure 6: Hela cells transfected with different MOI of HL-1 and HSV(17).
Figure 7: U251 cells transfected with different MOI of HL-1 and HSV(17).
Figure 8: Vero cells transfected with different MOI of HL-1 and HSV(17).
Figure 9: IC50 comparison of HL-1 and HSV(17) respectively transfecting different cells.
Figure 10: Measurement results of IC50 of CCTCC:V201810 virus strain and control virus strain 17 with ICP34.5 and ICP47 genes knockout transfecting different cells.
Figure 11: Measurement results of IC50 of CCTCC: V201810 virus strain and control virus strain 17 expressing IL-12 exogenous gene transfecting tumor cells.
Figure 12: Measurement results of IC50 of the virus strain HL-1 recombinant virus and virus strain 17 recombinant virus transfecting tumor cells after the insertion of IL-12 gene and anti-PD-1 monoclonal antibody gene.
Figure 13: Showing the comparison of the transfection ability of strain HL-1 and 17 recombinant viruses with ICP34.5 and ICP4 genes knockout on nerve cells.
Figure 14: Showing the comparison of the transfection ability of strain HL-1 and 17 recombinant viruses with ICP34.5, ICP4 and ICP27 genes knockout on nerve cells.
Figure 15: Showing the transfection of human fetal liver diploid cells and expression of exogenous genes in hepatocytes by strain HL-1 and 17 recombinant viruses with ICP34.5 and ICP4 genes knockout.
Figure 16: Showing transfection of human fetal liver diploid cells and expression of exogenous genes in hepatocytes by strain HL-1 and 17 recombinant viruses with ICP34.5, ICP4 and ICP27 genes knockout.
Figure 17: Showing the effect of administration of HSV-FVIII on APTT at 48 h, 72 h and 120 h.
Figure 18: Showing the effect of HL1-ENK on behavior and activity scores of arthritic chronic pain rats, demonstrating the significant analgesic effect of HL1-ENK on arthritic chronic pain rats.

### DETAIL DESCRIPTION OF THE APPLICATION

The present application relates to a non-replicating herpes simplex virus with improved performance compared to a laboratory model virus strain and its use as a vector. The term "oncolytic virus" in this application refers to a class of viruses that infect and kill cancer cells by destroying the infected cancer cells through oncolysis and releasing new infectious virus particles or virosomes that destroy the remaining cancer cells. These viruses are named for their oncolysis.

"Herpes simplex virus (HSV)", because of its deep research base and relative harmlessness in its natural state, was one of the first viruses chosen to selectively attack cancer cells (oncolytic virus). When the virus of the present application is a herpes simplex virus, the virus may be derived from, for example, an HSV-1 virus strain or an HSV-2 virus strain or a derivative virus strain thereof, preferably HSV-1.

Some embodiments of the present application relate to a virus strain with a CCTCC deposit number of V201810 (CCTCC: V201810). In this application, the virus strain is referred to as HSV-1 virus strain HL-1, or simply strain HL-1, HL-1, or HL1.

Some embodiments of the present application relate to a derivative virus strain of a HSV-1 strain, such as a virus strain with at least 70%, more preferably at least 80%, 85%, 90%, 95%, 98% sequence homology to the virus strain with a CCTCC deposit number of V201810 (CCTCC: V201810).

The term "herpes simplex virus vector" refers to a herpes simplex virus carrying an exogenous gene.

Plaque forming unit (pfu) refers to the number of viruses corresponding to the formation of plaques (phagocytic spots) on a monolayer culture of animal cells.

"Immune checkpoint blocking (inhibitory) antibody" or "immune checkpoint blocking (inhibitory) monoclonal antibody" refers to a monoclonal antibody that inhibits or blocks an inhibitory immune checkpoint molecule. "Immune checkpoint stimulating (agonistic) antibody" or "immune checkpoint stimulating (agonistic) monoclonal antibody" refers to a monoclonal antibody that stimulates or agonizes a stimulatory immune checkpoint molecule. Immune checkpoints are the regulators of the immune system, their functions are manifested by enhancing the immune system's ability to clear foreign bodies or by preventing the immune system from attacking cells indiscriminately, thus being very essential for immune regulation. Immune checkpoints are divided into inhibitory checkpoint molecules and stimulatory checkpoint molecules. Suppressive checkpoint molecules include but are not limited to: A2AR, B7-H3 (CD276), B7-H4, BTLA (CD272), CTLA-4 (CD152), IDO, KIR, LAG3, NOX2, PD-1, TIM3, VISTA, CD47; stimulatory checkpoint molecules include but are not limited to: CD27, CD40 OX40, GITR, CD137, CD28, and ICOS. Inhibitory checkpoint molecules and stimulatory checkpoint molecules are targets for cancer immunotherapy because they may be used in many types of cancer.

### 1. Herpes simplex virus wild strain HL-1 screened in this application

The present application relates in a first aspect to a herpes simplex virus, which is a herpes simplex virus type I (HSV-1) with a deposit number of CCTCC NO: V201810. The herpes simplex virus type I (HSV-1) has one or more properties selected from the group consisting of: (a) compared to a reference laboratory strain, having an enhanced ability to infect tumor cells; (b) compared to a reference laboratory strain, having an enhanced ability to replicate within tumor cells; (c) compared to a reference laboratory strain, having an enhanced ability to kill tumor cells; (d) after modification, the herpes simplex virus has an enhanced ability to infect tumor cells compared to a reference laboratory strain with equivalent modification; (e) after modification, the herpes simplex virus has an enhanced ability to replicate within tumor cells compared to a reference laboratory strain with equivalent modification; and (f) after modification, the herpes simplex virus has an enhanced ability to kill tumor cells compared to a reference laboratory strain with equivalent modification. In some embodiments, the present application relates to an attenuated variant of herpes simplex virus type 1 with a deposit number of CCTCC NO: V201810.

The virus strain of the application is a "non-laboratory" strain, which may be referred to as a "clinical" strain. Those skilled in the art can easily distinguish between laboratory and non-laboratory or clinical strains. The key difference between laboratory and non-laboratory strains is that the commonly used laboratory strains at present have been maintained in culture for a considerable period of time. To grow and maintain the virus, suitable cells are infected with the virus, the virus replicates within the cells, and then the virus is harvested; then again fresh cells are infected. This process constitutes a cycle of continuous passages. In the case of HSV, each such cycle may take, for example, a few days, and this continuous passage may lead to changes in the characteristics of the virus strain, such as the occurrence of selection for characteristics that favor growth in culture (e.g., rapid replication), rather than selection for characteristics that favor practical applications.

The virus strain of the present application is a non-laboratory strain because it is derived from a most recent isolate from an infected individual. The virus strain of the present application has been modified to eliminate virulence but retains essentially the desirable characteristics of the original clinical isolate from which it is derived.

The virus of the present application is capable of effectively infecting human target cells. Such virus is freshly isolated from an infected individual and then screened based on the desired ability to enhance replication *in vitro* and/or *in vivo* in tumor cells and/or other cells compared to standard laboratory strains. Such virus with improved properties compared to a laboratory virus strain is the virus of the present application. Identified virus with such desired improved characteristics can then be engineered by mutation of suitable genes so that it can selectively kill tumor cells. Or it is mutated so that it can deliver genes to target tissues with reduced toxic effects in non-oncolytic applications. This modified virus is also the virus of the present application.

The virus of the present application has a greater ability to infect any tumor cell or replicate therein, kill tumor cells, or spread intercellularly in tissues than a reference laboratory strain with equivalent modification. Preferably, this greater ability is a statistically significant greater ability. For example, in accordance with the present application, the ability of a virus strain of the present application may be at most 1-fold, 1.5-fold, 2-fold, 3-fold, 4-fold, 5-fold, 6-fold, 7-fold, 8-fold, 9-fold, 10-fold, 15-fold, 20-fold, 50-fold or 100-fold the ability of the reference strain in terms of the characteristics to be measured.

The virus of the present application has (i.e., retain) substantially the ability of its unmodified clinical precursor strain. For example, in the case of an oncolytic virus planned for tumor treatment, the virus strain of the present application preferably has substantially the ability of its unmodified clinical precursor strain to infect any tumor cell or replicate therein, kill tumor cells, or spread intercellularly in tissues.

### 2. Modified oncolytic virus of the present application

The present application covers modified viruses in which the altered viral region can either be eliminated (completely or partially) or become non-functional, or be replaced by other sequences, in particular by exogenous gene sequences. One or more genes can be made non-functional and one or more exogenous genes can be inserted.

In some embodiments, the viruses of the present application are modified non-laboratory oncolytic viruses. These viruses can be used in an oncolysis therapy to treat cancer. Such kind of viruses infect tumor cells and replicate within the tumor cells and subsequently kill the tumor cells. Thus, such kind of viruses are replication-competent viruses. Preferably, they have the ability to selectively replicate within the tumor cells. This means that they replicate within tumor cells but not within non-tumor cells, or that they replicate more efficiently within tumor cells than within non-tumor cells. The determination of selective replication capacity can be performed as described herein by the assays for determining replication capacity and tumor cell killing capacity. Also, the viruses of this application can be modified to lose their ability to replicate in any cell, including tumor cells, but still have the ability to infect cells, including tumor cells. The modified viruses exert their tumor-killing effects by intracellular expression of the carried tumor therapeutic genes. In cancer therapy, oncolytic viruses with the ability to replicate in tumor cells are preferably used for treatment.

Preferably, the oncolytic viruses of the present application have a (preferably statistically significant) greater ability to infect tumor cells or replicate within tumor cells, kill tumor cells, or spread intercellularly in tissues than a reference laboratory strain with the same modification.

The ability of the virus to infect tumor cells can be quantified by measuring the dose of virus required to infect a certain percentage of cells (e.g., 50% or 80% of cells). The ability to replicate within tumor cells can be determined by measuring the growth of the virus in cells, for example by measuring the growth of the virus within the cell over a time period of 6 hours, 12 hours, 24 hours, 36 hours, 48 hours, or 72 hours or longer. The ability of the virus to kill tumor cells can be quantified by counting the number of live cells retained at a given time point and MOI (multiplicity of infection) for a given cell type over a defined time period. Cells or combinations of cells from embodiments of this application as well as other tumor cell types can be used. To count the number of live cells retained at a given time point, the number of cells without trypan blue (i.e., live cells) are counted. It can also be quantified by analysis using flow cytometry (FACS), MTT, CCK-8 or CTG method. Tumor cell killing capacity can also be measured *in vivo,* for example by measuring the reduction of tumor volume due to a specific virus.

To characterize the virus of the present application, it is generally preferable to use a standard laboratory reference strain for comparison. Any suitable standard laboratory reference strain can be used. In the case of HSV, it is preferable to use one or more of HSV-1 strain 17, HSV-1 strain F or HSV-1 strain KOS. The reference strain typically have modifications, such as gene deletions and/or exogenous gene insertions, equivalent to those of the strain to be tested in the present application. For example, in the case of HSV strains, if the genes encoding ICP34.5, ICP6 and/or thymidine kinase (TK) have been made non-functional in the virus of the present application, they are also made non-functional in the reference strain.

In embodiments of the present application, the reference strain is a laboratory standard model strain HSV-1 virus strain 17, or known as HSV-1 virus strain 17, HSV(17), virus strain 17, strain 17.

This application relates to the human herpesvirus type I strain HL-1 with a deposit number CCTCC NO: V201810, deposited at the China Center for Type Culture Collection (CCTCC) (address: Wuhan University, Wuhan, China) on July 30, 2019, for the purposes of patent procedure.

In this application, herpesvirus type I (type 1), type I (type 1) herpesvirus, herpes simplex virus type I (type 1), type I (type 1) herpes simplex virus, and HSV-1 have the same meaning and can be used interchangeably. Human herpesvirus type I strain HL-1 can be abbreviated as strain HL-1, strain HL1, HL-1 or HL1 in this application.

Herpes simplex virus (HSV) can be either used as a gene delivery vector in the nervous system and other systems, or used in oncolytic therapy for cancer treatment. In both applications, however, the virus must be made defective so that it is no longer pathogenic but can still enter cells and perform the desired function.

The HSV-1 genome has many genes that can be knocked out or mutated to confer safety and/or tumor targeting specificity. For example, one of the common features that distinguishes cancer cells from most normal cells (post mitotic) is the continued proliferation of cancer cells and the consequent maintenance of sufficient nucleotides for DNA replication. HSV-1 contains genes involved in nucleotide metabolism (thymidine kinase, ribonucleotide reductase and uracil DNA glycosylase, etc.) allowing viral replication in non-dividing cells that lack sufficient nucleotides. Knocking out or mutating these genes can confer the virus specificity for dividing cells (tumor cells) and also often attenuates the pathogenicity of the virus. In addition, cells have multiple mechanisms to detect and inactivate invading viruses, and a portion of the viral genes (ICP34.5, ICP0 and UL56, etc.) can evade or block the cell's antiviral mechanisms, and knockout or mutation of this portion of the genes can likewise confer the virus specificity for dividing cells (tumor cells).

In some embodiments, the HSV strain of the application is modified so that it lacks one or more of the ICP34.5 gene, ICP6 gene, ICP0 gene, ICP47 gene, US3 gene, UL56 gene, functional VP16 gene, VHS gene, UNG gene, glycoprotein H gene, and thymidine kinase gene. In some embodiments, the virus lacks the ICP34.5 gene. In some embodiments, the virus can be further modified so that it lacks the ICP47 gene. In some embodiments, one or more of the ICP34.5, ICP47, Us3, ICP0, UL56 genes are mutated to prepare an oncolytic herpes simplex virus type 1 (oHSV-1).

For the oncolytic therapy to treat cancer (which may also include the delivery of genes that enhance the effect of the therapy), many mutations in HSV have been identified (Peters C. Designing Herpes Viruses as Oncolytics. Molecular Therapy Oncolytics, 2015.), these mutations in HSV still allow the virus to replicate in cultures or within actively dividing cells *in vivo* (e.g., within tumors), but prevent its efficient replication in normal tissues. Such mutations include disruption of the genes encoding ICP34.5, ICP6, and thymidine kinase. To date, of these mutant viruses, those with ICP34.5 mutation or ICP34.5 mutation and, for example, ICP6 mutation have shown the most favorable safety profile. Viruses lacking only ICP34.5 have been shown to replicate in many tumor cell types *in vitro* and selectively replicate within mouse brain tumors without harming surrounding tissues.

Any other viruses with genetic deletions/mutations that provide oncolytic properties (i.e., selective replication within tumors compared to surrounding tissues) are also viruses covered by this application.

By using techniques known in the art and/or described herein, exogenous genes may be inserted into such kind of viruses of the present application, for example the virus with a CCTCC deposit number of V201810 (CCTCC: V201810). In oncolytic viruses, the exogenous gene is usually a gene that enhances the ability of the virus to fight tumors. Thus, any gene that confers anti-tumor properties to the virus can be inserted. Specifically, the exogenous gene may be a gene capable of improving the immune response against tumor cells in a beneficial manner, in particular an immunostimulatory polypeptide.

### 3. Modified non-replicating virus of this application

The present application relates to genetically modified herpes simplex viruses and their use as vectors in non-oncolystic applications. The genetically modified herpes simplex viruses of the present application used as vectors in non-oncolytic applications can be mutated such that the expression of virus regulatory immediate early genes is minimized. Thus, the genes encoding ICP4, ICP27, ICP22 and/or ICP0 can be inactivated or absent, either alone or in combination, or mutations in the transactivation protein vmw65 of the viriosome include blocking/reducing its transactivation capacity. In particularly preferred embodiments for non-oncolytic applications, genes encoding ICP27, ICP0, and ICP4 are absent (with or without additional ICP22 and/or ICP47 absent/inactivation), or ICP27 and ICP4 are absent, plus inactivation in vmw65, or ICP4 is absent, plus inactivation in vmw65.

Some embodiments of the present application relate to herpes simplex virus vectors that deliver exogenous genes to the nervous system. Viruses in accordance with this embodiment of the present application are commonly more capable of infecting neurons, transmissing intercellularly in nerve tissues, or transporting in axon than reference laboratory strains with equivalent modifications. In these embodiments, the virus of the present application is modified so that it lacks one, two, three, or all of the genes encoding functional ICP27, the genes encoding functional ICP4, the genes encoding functional ICP0, or the genes encoding functional ICP22. In some embodiments, the viruses of the present application lack both the functional ICP4 coding gene and the functional ICP27 coding gene, and the viruses of the present application have an inactivating mutation in the vmw65 coding gene that eliminates its transcriptional activation activity. Such viruses can be used to treat peripheral nervous system diseases or central nervous system diseases. In the case of central nervous system diseases, it is particularly preferred to make at least two immediate early genes selected from ICP0, ICP4, ICP22 and ICP27 non-functional. To improve the safety of the modified virus, the virus of the present application can also be modified on one or more of the ICP34.5 gene, ICP6 gene, ICP0 gene, ICP47 gene, US3 gene, UL56 gene, functional VP16 gene, VHS gene, UNG gene, glycoprotein H gene, and thymidine kinase gene. The exogenous gene insertion site can be any one or more of the above modification sites, or it can be the latent infection region (LAT) of the herpes simplex virus. When the herpes simplex virus enters the latent state, all genes are at the dormant state except for the genes in the LAT region, which are at the active state, and insertion of the exogenous genes into the LAT region contributes to the long-term expression of the genes. Therefore, when the virus of the present application is used in non-replicating gene therapy as a vector, it is preferable to insert exogenous genes into the LAT region.

The non-replicating viruses of the present application can be used to deliver genes to individuals in need of gene therapy. Specifically, the herpes simplex virus of the present application can be used to treat central nervous system diseases or peripheral nervous system diseases, used for treat or prevent the central nervous system diseases including neurodegenerative diseases. In some embodiments, the central nervous system diseases for treatment or prevention are stroke, Parkinson's disease, Alzheimer's disease, amyotrophic lateral sclerosis, and Huntington's disease. Preferred peripheral nervous system diseases for treatment or prevention include motor neuron disease, chronic pain, and peripheral nerve injury. In some embodiments, the virus of the present application is administered to a subject suffering from pain, degenerative disease, or nerve injury to improve the condition of the subject, for example by reducing the severity of pain, slowing the degeneration of nerve tissues, or promoting nerve regeneration growth. In some embodiments, the herpes simplex virus of the present application can be used to treat hematological system diseases. Specifically, the hematological system diseases include hemophilia.

### 4. Pharmaceutical composition, article of manufacture and uses thereof

The oncolytic viruses of the present application can be used in the preparation of a medicament for the treatment of cancer. Specifically, the oncolytic virus preparations of the present application can be used in the treatment of cancer, for example, by direct intra-tumor injection. The oncolytic virus preparations of the present application can be used to treat any solid tumor in mammals, preferably humans. For example, the virus of the present application can be administered to subjects suffering from a disease selected from the group consisting of: liver cancer, melanoma, glioma, sarcoma, lung cancer, colorectal cancer, head and neck tumors, breast cancer, renal cell cancer, ovarian cancer, cervical cancer, prostate cancer, gastric cancer, lymphoma, pancreatic cancer, and bladder cancer.

In some embodiments, the present application relates to a recombinant oncolytic virus or referred to as a modified oncolytic virus, characterized in that the recombinant oncolytic virus or modified oncolytic virus is derived from a herpes simplex virus type I with a CCTCC deposit number of V201810 (CCTCC NO: V201810), wherein the CCTCC: V201810 virus is genetically modified so that one or more of the ICP34.5 gene, ICP6 gene, ICP0 gene, ICP47 gene, US3 gene, UL56 gene, functional VP16 gene, VHS gene, UNG gene, glycoprotein H gene, and thymidine kinase gene are knocked out, such that exogenous genes are inserted into one or more positions of the knockout genes. The exogenous genes are for example the genes encoding cytokines stimulating an immune response or antibody polypeptides capable of promoting an immune response, for example the cytokine polypeptide stimulating an immune response is granulocyte macrophage colony stimulating factor (GMCSF), IL-2, IL12, IFN-γ or TNF-α, and the antibody polypeptide capable of promoting immune response is an immune checkpoint antibody.

The recombinant oncolytic viruses of the present application or referred to as modified oncolytic viruses can be used in the preparation of a medicament for the treatment of cancer. Specifically, the oncolytic virus preparations of the present application can be used in the treatment of cancer, for example by direct intra-tumor injection. The oncolytic virus preparation of the present application can be used to treat any solid tumor in mammals, preferably humans. For example, the virus of the present application can be administered to subjects with a disease selected from the group consisting of: liver cancer, melanoma, glioma, sarcoma, lung cancer, colorectal cancer, head and neck tumors, breast cancer, renal cell carcinoma, ovarian cancer, cervical cancer, prostate cancer, gastric cancer, lymphoma, pancreatic cancer, and bladder cancer.

The present application relates to use of non-replicating viruses in gene delivery to individuals in need of gene therapy. In some embodiments, the genetically modified herpes simplex virus vectors of the present application can be used to treat central nervous system diseases or peripheral nervous system diseases. The central nervous system diseases to be treated or prevented include neurodegenerative diseases. In some embodiments, the central nervous system diseases to be treated are stroke, Parkinson's disease, Alzheimer's disease, amyotrophic lateral sclerosis, or Huntington's disease. Peripheral nervous system diseases to be treated or prevented include motor neuron disease, chronic pain, and peripheral nerve injury. In some embodiments, the viruses of the present application are administered to subjects suffering from pain, degenerative disease, or nerve injury to improve the condition of the subjects, for example by reducing the severity of pain, slowing the degeneration of nerve tissues, or promoting nerve regeneration growth. In some embodiments, the herpes simplex viruses of the present application can be used to treat hematological system diseases. Specifically, hematological system diseases include hemophilia.

Some embodiments of the present application involve pharmaceutical a composition comprising the virus of the present application and a pharmaceutically acceptable carrier or diluent. Suitable carriers and diluents are known in the art and include, but are not limited to, phosphate buffered salt solutions.

In some embodiments, the present application relates to a article of manufacture or a kit comprising a vial containing the pharmaceutical composition described above and a package insert, and the package insert includes instructions for how to use the article of manufacture or kit to treat a disease.

The pharmaceutical composition of the present application may be injected directly into a target tissue, such as a cancer tissue, for oncolytic therapy treatment and/or gene delivery into a cell for therapeutic purposes. The pharmaceutical composition of the present application comprising viruses or recombinant viruses are administered in a virus dosage range of 10⁴-10¹² pfu/ml, preferably 10⁵-10⁸ pfu/ml, more preferably 10⁶-10⁸ pfu/ml. For oncolytic or non-oncolytic treatment, during injection, the injection dosage of the pharmaceutical composition consisting essentially of the virus and a pharmaceutically acceptable suitable carrier or diluent is commonly up to 10 ml, commonly 1-5 ml, preferably 1-3 ml. However, for some oncolytic therapy applications, a larger volume of more than 10 ml may also be used, depending on the tumor and the position of inoculation.

Those skilled in the art can easily determine the optimal route of administration and dose based on the condition of the subject and the disease. The dose can be determined according to various parameters, in particular according to the age, weight and condition of the patient to be treated, the severity of the disease or condition and the route of administration. The preferred route of administration for cancer patients is direct injection into the tumor. It is also possible to systematically administer the virus, for example intravenously, or to administer the virus by injection into the blood vessels that supply blood to the tumor. The most suitable route of administration will depend on the location and size of the tumor. The dose can be determined according to various parameters, in particular according to the location of the tumor, the size of the tumor, the age, weight and disease of the patient to be treated and the route of administration.

### EXAMPLE

### Example 1. Acquisition of strain HL-1 virus

a) Collection of virus
   Sixty-three healthy volunteers with recurrent herpes labialis were recruited and the herpes fluid was collected aseptically.
b) Screening of the virus
   The purpose of this part is to test and screen primary clinical isolates of HSV-1, for selecting the virus strains with the greatest oncolytic capacity.

Five to eight virus strains were simultaneously used each time for the experiment. HSV-1 virus strain 17 was used as a control. Cells in six-well plates were infected with virus strains at the same MOI and tested in parallel. And the virus-induced cytopathic effect (CPE) phenomenon was observed 48 h after infection and the number of plaques after crystal violet staining was evaluated.

CPE should be induced by typical HSV-1 infection, with cells rounded and gradually shed from the infection center to form plaques. Virus strains with significant CPE phenomenon and biggest number of plaques after staining were evaluated.

The virus strain with the best oncolytic effect was evaluated and selected as strain HL-1 by this method.

### c) Biological characterization of the virus strain HL-1

### 1) Electron microscopic observation

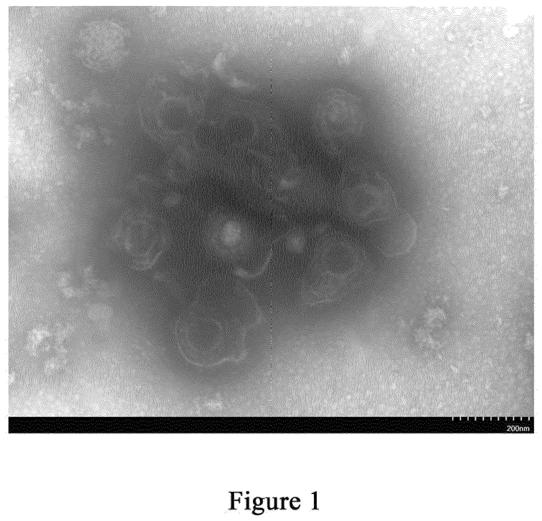

Cells were infected with the strain HL-1 virus and cell debris was removed, and the cells were observed under a 25000x electron microscope. The figure showed that the virus had a typical HSV-1 appearance, and the four components, genomic DNA, capsid, tegument protein and envelope could be observed (see Figure 1).

### (2) Analysis of the genomic DNA sequencing result of the virus strain HL-1

The genomic DNA of herpes simplex virus type I (HSV-1) strain HL-1 was analyzed by third generation sequencing performed by Shanghai OE Biotech, and the virus genome sequences of Strain HL-1 and the standard model HSV-1 virus strain 17 (NC_001806.2) were compared. The results showed that there were more than 1700 base differences in the genome sequences of the two, and there were great differences in the amino acid sequences of many important encoding genes, including membrane proteins and immediate early genes. The comparison results are shown in Tables 1 and 2 below.

**Table 1: Comparison results of membrane protein amino acid sequences of strain HL-1 and strain 17**

| Membrane protein | Peptide chain length (aa) | Amino acid difference | Percentage of amino acid difference |
|---|---|---|---|
| gB | 904 | 4 | 0.44% |
| gC | 511 | 1 | 0.20% |
| gD | 394 | 4 | 1.02% |
| gE | 550 | 2 | 0.36% |
| gG | 238/239 | 13 | 5.46% |
| gH | 838 | 4 | 0.48% |
| gI | 390 | 13 | 3.33% |
| gJ | 92 | 2 | 2.17% |
| gK | 338 | 0 | 0.00% |
| gL | 224 | 3 | 1.34% |
| gM | 473 | 6 | 1.27% |
| gN | 91 | 2 | 2.20% |

**Table 2. Comparison results of amino acid sequences of important genes in strain HL-1 and strain 17**

| Gene name | Peptide chain length (aa) | Amino acid difference | Percentage of amino acid difference |
|---|---|---|---|
| ICP0 | 775/774 | 2 | 0.26% |
| ICP4 | 1293/1298 | 22 | 1.70% |
| ICP6 | 1137 | 6 | 0.53% |
| ICP22 | 420 | 8 | 1.90% |
| ICP27 | 512 | 1 | 0.20% |
| ICP47 | 88 | 1 | 1.14% |
| ICP34.5 | 256/248 | 16 | 6.25% |

The amino acid sequence differences of gD, gG, gI, gJ, gL, gM, and gN in the envelope proteins between strain HL-1 and strain 17 were more than 1%, especially the amino acid sequence differences of gG and gI were more than 3%, wherein that of gG reaching 5.46%. The membrane proteins of the herpes simplex virus envelope were associated with virus invasion, release, and direct intercellular transmission, suggesting differences in invasiveness between strains HL-1 and 17.

Comparison of the amino acid sequences of the early genes ICP0, ICP4, ICP22, ICP27, ICP47 and the important genes ICP6 and ICP34.5 revealed that the sequences of the early genes ICP4, ICP22 and ICP47 differed significantly between strain HL-1 and strain 17, with amino acid sequence differences exceeding 1%. As the immediate early genes of HSV-1, ICP4 and ICP22 can stimulate the expression of early genes and DNA synthesis of the virus, and induce the expression of late genes. They are the key factors for HSV-1 gene expression which causes infection. ICP34.5 is also one of the genes that differed significantly between strain HL-1 and strain 17, with an amino acid difference of 6.25%. ICP34.5 is an important neurotoxic factor of HSV-1 virus and plays a key role in virus replication and pathogenicity.

### d) Deposit of the virus strain HL-1

The virus strain HL-1 was deposited for the purposes of patent procedure on July 30, 2019, at the China Center for Type Culture Collection (CCTCC) (Address: Wuhan University, Wuhan, China) under a deposit number of CCTCC NO: V201810, with the classification designation: "Human herpesvirus type I strain HL-1 ".

### Example 2 Comparison of the oncolytic effects of wild strain HL-1 and strain 17

This Example provides comparative replication experiments and results of strain HL-1 and strain 17 on different cells and their ability to transfect tumor cells. The experimental cells include: Hep G2 human liver cancer cells, A-375 human skin cancer cells, A549 human lung cancer cells, MCF7 human breast cancer cells, HeLa human cervical cancer cells, U251 human glioma cells, and Vero monkey kidney cells.

The experimental methods were as follows:
1. Microscopic observation after transfection of different cells with HL-1 and HSV(17) respectively
   The above cells in logarithmic phase were digested and passaged into six-well plates for culture. 24 hours later, the old medium was discarded and 2 ml of medium was added to each well. The viruses at different MOI was for transfection. After adding the virus, it was placed in a 37°C carbon dioxide incubator for 48 hours, and then taken out for observation under a microscope, and photographed and recorded.
2. IC50 measurement of HL-1 and HSV(17) transfected in different cells respectively
   Cells in logarithmic phase were digested and passaged into 96-well plates. 24 hours later, the old medium was discarded and HL-1 and HSV(17) viruses at 9 dilution gradients were added respectively, 100 µl/well, making the MOI 20, 5, 1.25, 0.3125, 0.0781, 0.0195, 0.0049, 0.0012 and 0.0003, and a blank control was set. After incubation at 37°C for 3 days, all liquid in the wells was discarded, 10% CCK-8 reagent (Cell Counting Kit-8, Dojindo) was added, 100 µl/well, and incubated at 37°C for 1 h. The OD values of the samples were measured at 450 nm, and the IC50 was calculated according to the fitted curve.

### Experiment Results:

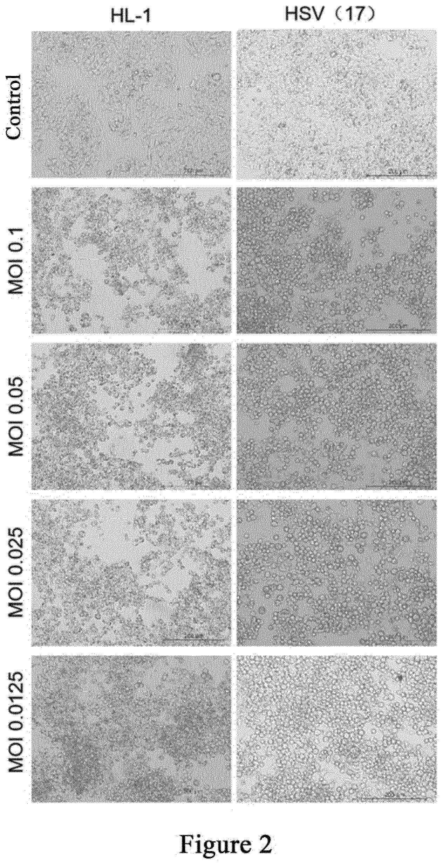

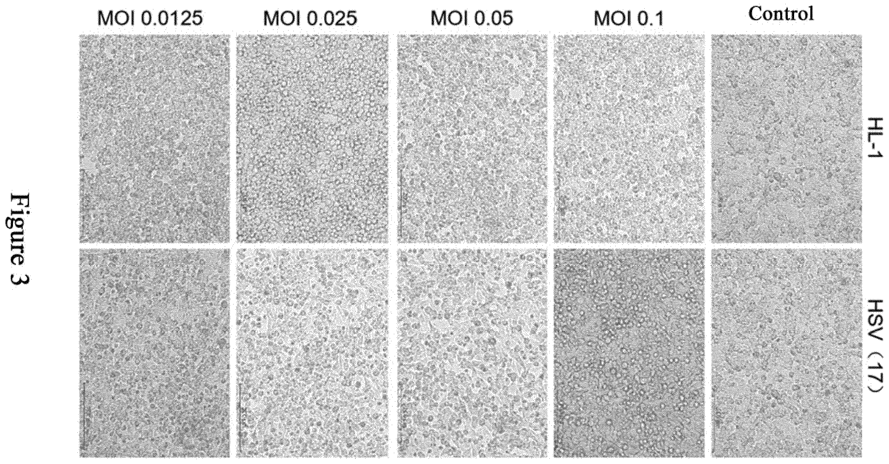

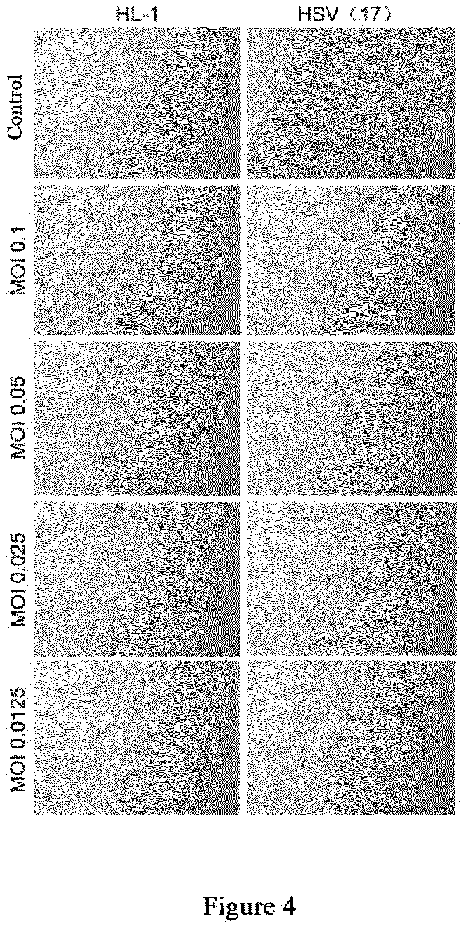

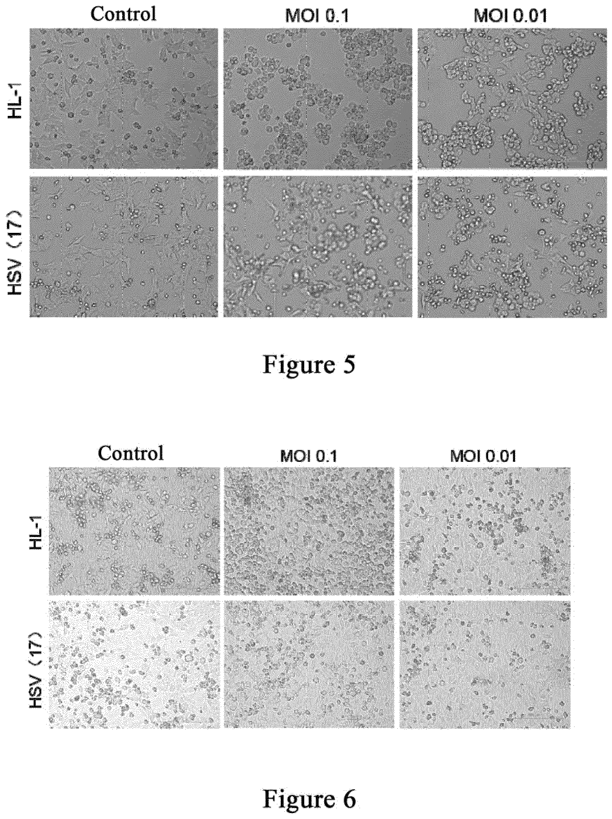

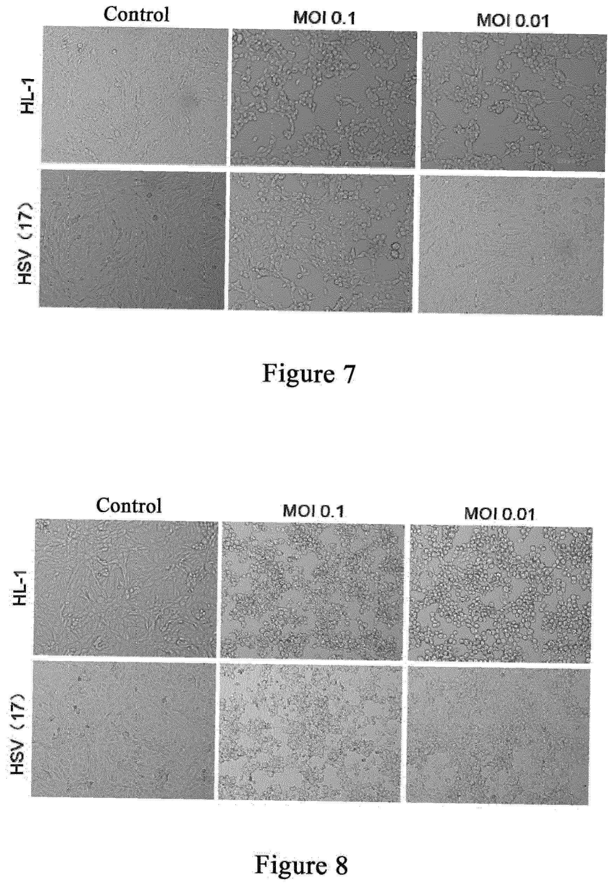

1. Microscopic observation after transfecting different cells with HL-1 and HSV (17) respectively
   1.1 Microscopic results of HL-1 and HSV(17) transfected HepG2 cells
      The microscopic observation result of HepG2 cells 48 hours after transfection with HL-1 and HSV(17) showed that the transfection efficiency of HL-1 was higher than that of HSV(17) under each of the same MOI conditions (see Figure 2).
   1.2 Microscopic results of HL-1 and HSV(17) transfected A375 cells
      The microscopic observation of A375 cells 48 hours after transfection with HL-1 and HSV(17) showed that the transfection efficiency of HL-1 was significantly higher than that of HSV(17) under each of the same MOI conditions. It could even be observed under the microscope that the morphology of HL-1 transfected cells was all rounded under the condition of transfection with virus MOI 0.1, while normal cells were still visible in HSV(17) transfected cells (see Figure 3).
   1.3 Microscopic results of HL-1 and HSV(17) transfected A549 cells
      The microscopic observation results of A549 cells 48 hours after transfection with HL-1 and HSV(17) showed that the transfection efficiency of HL-1 was significantly higher than that of HSV(17) under each of the same MOI conditions. It could be observed under the microscope that the number of HL-1 transfected cells was significantly more than that of HSV(17) transfected cells, and almost no normal cells were observed at MOI 0.1, while normal cells were still visible in HSV(17) transfected cells, and the number increased incrementally with the decrease of MOI (see Figure 4).
   1.4 Microscopic results of HL-1 and HSV(17) transfected MCF-7 cells
      Based on the experience of comparing the effect of virus transfected cells in the above experiments, for the subsequent experiments only two virus concentrations, MOI 0.1 and MOI 0.01, were selected for comparison.
      The microscopic observation of MCF-7 cells 48 hours after transfection with HL-1 and HSV(17) showed that the transfection efficiency of HL-1 was significantly higher than that of HSV(17). The cell morphology of HL-1 transfected MCF-7 cells was all rounded under the condition of MOI 0.1, while some normal cells were still present in HSV(17) transfected MCF-7 cells (see Figure 5).
   1.5 Microscopic results of HL-1 and HSV(17) transfected Hela cells
      Microscopic observation of Hela cells 48 hours after transfection with HL-1 and HSV(17) showed that the transfection efficiency of HL-1 was significantly higher than that of HSV(17) at MOI 0.1. The morphology of HL-1 transfected Hela cells was mostly rounded under the condition of MOI 0.1, while HSV(17) transfected Hela cells still had some normal cells (see Figure 6).
   1.6 Microscopic results of HL-1 and HSV(17) transfected U251 cells
      The microscopic observation of U251 cells 48 hours after transfection with HL-1 and HSV(17) showed that the transfection efficiency of HL-1 was significantly higher than that of HSV(17), and the morphology of HL-1 transfected U251 cells was mostly rounded under the condition of MOI 0.01, while only a few cells of HSV(17) transfected U251 cells were rounded by the virus transfection (see Figure 7).
   1.7 Microscopic results of HL-1 and HSV(17) transfected Vero cells
      Vero cells are non-tumor cells, a sensitive cell line for HSV virus, and one of the commonly used production cells, so comparing the infection of Vero cells by HL-1 and HSV(17) is of great importance for production capacity.
      Microscopic observation of Vero cells 48 hours after transfection with HL-1 and HSV(17) showed that the transfection efficiency of HL-1 was significantly higher than that of HSV(17) under the condition of both MOI 0.1 and 0.01. The cell morphology of HL-1 transfected Vero cells was all rounded under the condition of virus MOI 0.1, while the HSV(17) transfected Vero cells still had some normal cells at under the condition of virus MOI 0.1 (see Figure 8).
2. IC50 measurements of HL-1 and HSV(17) respectively transfecting different cells

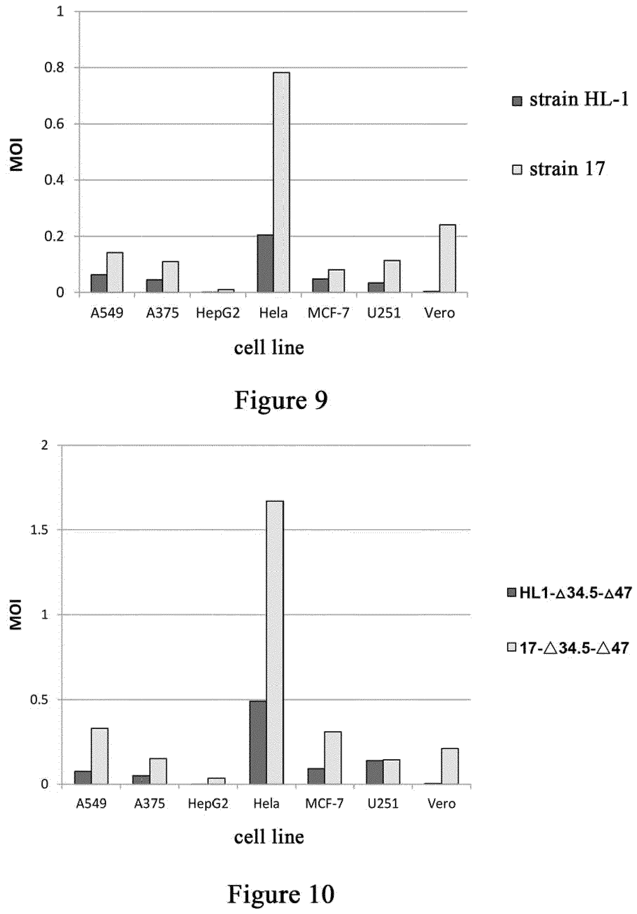

The comparison of the IC50 of HL-1 and HSV(17) transfecting different cells is shown in Figure 9. The results showed that the IC50 (MOI) of HL-1 was smaller than that of HSV(17), indicating that HL-1 had a stronger ability to kill cells than HSV(17). The purpose of this example is to compare the differences in the ability of the two virus strains HL-1 and HSV (17) to transfect tumor cells. HL-1 and HSV (17) were used to transfect different tumor cells (HepG2, A375, A549, MCF-7, Hela and U251) as well as normal passaged Vero cells, and the results observed under the microscope 48 hours after transfection of the cells showed that although the sensitivity of each cell type to the virus was different, the transfection efficiency of HL-1 was significantly higher than that of HSV (17) at the same MOI. Further, the IC50 values of HL-1 and HSV(17) transfecting different cells were determined by CCK-8 method, and the results showed that the IC50 values of HL-1 when transfecting cells were all smaller than those of HSV(17). In conclusion, compared with the virus strain HSV(17), the virus strain HL-1 had better infection efficiency and replication ability in cells, and stronger oncolytic effect.

### Example 3 Comparison of the oncolytic effects of HSV (HL-1) recombinant virus and HSV (17) recombinant virus

The ICP34.5 gene and ICP47 gene were knocked out from the virus strain with a CCTCC deposit number of V201810 (CCTCC: 201810) of the present application and the control virus strain 17 according to the method documented in patent application No. CN1654667A, and difference in tumor cell transfection ability of the recombinant viruses HL1-△34.5-△47 and 17-△34.5-△47 after gene knockout were compared. The experimental cells included Hep G2 human liver cancer cells, A375 human skin cancer cells, A549 human lung cancer cells, MCF7 human breast cancer cells, HeLa human cervical cancer cells, U251 human glioma cells, and Vero monkey kidney cells.

Cells in logarithmic phase were digested and passaged into 96-well plates. 24 hours later, the old medium was discarded and different viruses at 9 dilution gradients were added, 100 µl/well, making MOI 20, 5, 1.25, 0.3125, 0.0781, 0.0195, 0.0049, 0.0012 and 0.0003, and a blank control was set up. After 3 days of incubation at 37°C, all the liquid in the wells was discarded, 10% CCK-8 reagent was added, 100 µl/well, and they were incubated for 1-2 hours at 37°C, the OD values of the samples were measured at 450 nm and the IC50 value was calculated according to the fitted curve.

The IC50 comparison results of the two recombinant viruses transfecting different cells are shown in Figure 10. The results showed that the IC50 values of HL1-△34.5-△47 were all smaller than those of 17-△34.5-△47 when transfecting cells, indicating that the virus strain HL-1 after recombination, had a better infection efficiency and replication ability in cells, and stronger oncolysis, than that of the recombinant virus of virus strain HSV(17), thus having a better application prospects as an oncolytic virus.

### Example 4 Oncolysis effects of virus strain HL-1 as a vector carrying exogenous genes on tumor cells

The purpose of this example is to compare the oncolysis effects of the HL-1 recombinant virus and the virus strain 17 recombinant virus carrying exogenous genes. The experimental cells include A375 human skin cancer cells, A549 human lung cancer cells, AGS human gastric cancer cells, AsPC-1 human pancreatic cancer cells, HeLa human cervical cancer cells, Hep G2 human liver cancer cells, HT-29 human colorectal cancer cells, MCF7 human breast cancer cells, PC-3 human prostate cancer cells, T24 human bladder cancer cells, U-2 OS human bone cancer cells and U-87 human glioma cells.

### 1. Comparison of oncolysis effects of oncolytic viruses expressing exogenous gene IL-12

The ICP34.5 gene and ICP47 gene were knocked out from the virus strain with a CCTCC deposit number of V201810 (CCTCC: V201810) of the present application and the control virus strain 17 according to the method of Example 3, and a synthetic chemically synthesized exogenous gene was inserted at the position where the ICP34.5 gene was knocked out. The genes were sequenced at Tsingke Biotechnology, Beijing and identified as correctly inserted into the herpes simplex virus vectors. The successfully constructed recombinant virus vectors were proliferated with the above cell lines at 37°C and 5% CO₂.

Cells in logarithmic phase were digested and passaged into 96-well plates. 24 hours later, the old medium was discarded and different viruses at 9 dilution gradients were added, 100 µl/well, making MOI 20, 5, 1.25, 0.3125, 0.0781, 0.0195, 0.0049, 0.0012 and 0.0003, and a blank control was set up. After 3 days of incubation at 37°C, all the liquid in the wells was discarded, 10% CCK-8 reagent was added, 100 µl/well, and they were incubated for 1-2h at 37°C, the OD values of the samples were measured at 450 nm, and the IC50 values were calculated according to the fitted curve.

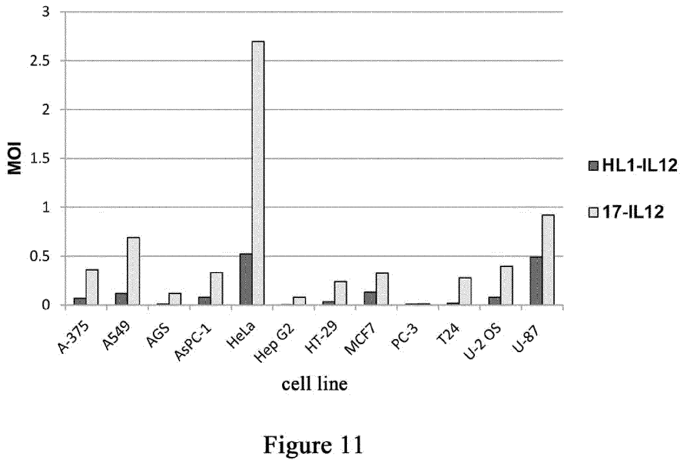

The results of IC50 measurement of different viruses transfecting tumor cells are shown in Figure 11. The experimental results showed the difference in the abilities of the HL-1 recombinant virus (HL1-IL12) and the strain 17 recombinant virus (17-IL12) to transfect tumor cells after inserting IL-12 gene. The IC50 values of the HL-1 recombinant virus inserted with IL-12 gene were smaller than those of the strain 17 recombinant virus inserted with IL-12 gene, indicating that the recombinant virus of virus strain HL-1 inserted with IL-12 gene had better infection efficiency and replication ability in tumor cells and stronger tumor oncolysis, and had better application prospects as oncolytic virus.

### 2. Oncolysis effects of the oncolytic virus introduced with exogenous gene IL-12 on melanoma mice

HL1-IL12 and HL1-mock (HL1-Δ34.5-Δ47) were constructed according to the methods of Examples 3 and 4 to assess the oncolysis effects of oncolytic viruses on melanoma mice. C57BL/6J mice were subcutaneously inoculated with B16F10 tumor cells to establish a homoplastic melanoma model. The control group was phosphate solution, and the treatment groups were oxaliplatin (10 mg/kg), tested oncolytic virus HL1-mock (10^7 pfu), HL1-IL12 (10^7 pfu), HL1-IL12 (10^6 pfu) and HL1-IL12 (10^5 pfu), respectively, 10 mice in each group; the control and tested oncolytic virus administration groups were administered intratumorally, and the oxaliplatin administration group was administered intraperitoneally.

Relative tumor inhibition rate TGI (%): TGI % = (1 - T/C) × 100%. T/C % is the relative tumor proliferation rate, i.e. the percentage value of the relative tumor volume in a treatment group to that in the control group at specific time points. T and C are the relative tumor volumes (RTVs) in the treatment and control groups at specific time points, respectively. RTV = tumor volume in treated animals/ tumor volume in the control group. Efficacy was evaluated according to the tumor inhibition rate (TGI).

Research results: By analyzing the mean tumor volume of mice on day 10 after administration, the tumor inhibition rate was 76% for HL1-IL12 (107 PFU), the tumor inhibition rate was 47% for HL1-IL12 (106 PFU), the tumor inhibition rate was 52% for HL1-mock (10⁷ PFU), and all the three had statistically significant differences in anti-tumor effects compared to that of the control group (P<0.05). There was no statistically significant difference (P < 0.05) in the antitumor effects of HL1-IL12 (10⁵ PFU) and oxaliplatin (10 mg/kg) single administration groups compared with that of the control group, and the tumor inhibition rates were 6% and 20%, respectively. All mice in the control group died on day 14, while three mice in the HL1-IL12 (10⁷ PFU) administration group had tumor disappearance and sustained survival. Except for the three mice mentioned above, all other mice in the administration group died on day 21.

The remaining three mice in the HL1-IL12 (10⁷ PFU) group had tumor disappearance and survived continuously till day 91. The mice were again inoculated with B16F10 tumor cells on the opposite side for re-challenge, and 5 C57BL/6J mice in the control group were inoculated with B16F10 tumor cells subcutaneously to observe the tumor growth.

After inoculation, tumors grew in the control group on day 10 and continued to increase in size, while no tumors grew in the re-challenged group, and continued observation still did not result in tumor growth, proving that the mice had tumor immunity after HL1-IL12 treatment.

### 3. Oncolysis effects of oncolytic viruses expressing exogenous genes GM-CSF and IL-2

The ICP34.5 gene and ICP47 gene were knocked out from the virus strain with a CCTCC deposit number of V201810 (CCTCC: V201810) of the present application and the control virus strain 17 according to the method of Example 3, and a synthetic chemically synthesized exogenous gene was inserted at the position where the ICP34.5 gene was knocked out. The exogenous genes included, from 5' end to 3' end, CMV promoter, a gene encoding GM-CSF (Gene ID: 12981), BGH PolyA, EF1α promoter, a gene encoding IL-2 (Gene ID: 16183) and TK PolyA. The above coding genes were correctly inserted into the herpes simplex virus vector by sequencing at Beijing Sunbiotech.

Animal models were constructed by subcutaneous inoculation of C57BL/6 mice with a murine-derived melanoma B16F10 cell line. Mice that were successfully modeled were selected and 9 groups were set up with 5 mice in each group. In the HL-1 recombinant virus treatment group, the HL-1 recombinant virus with exogenous genes GM-CSF and IL-2 was administered; in the virus strain 17 recombinant virus treatment group, the virus strain 17 recombinant virus with exogenous genes GM-CSF and IL-2 was administered; in the HSV-mock group, an oncolytic virus with no exogenous inserted and ICP34.5 gene and ICP47 gene knocked out was administered; in the negative control group, PBS was administered. The total pfu administered to each group was 10⁶ pfu. The relative tumor inhibition rate after 14 days of administration was used as the basis for judgment.

Relative tumor inhibition rate TGI (%): TGI % = (1 - T/C) × 100%. T/C % is the relative tumor proliferation rate, i.e. the percentage value of the relative tumor volume in a treatment group to that in the control group at specific time points. T and C are the relative tumor volumes (RTVs) in the treatment and control groups at specific time points, respectively. RTV = tumor volume in treated animals/ tumor volume in the control group.

Experiment results: The relative tumor inhibition rate was 79% in the HL-1 recombinant virus treatment group; the relative tumor inhibition rate was 58% in the virus strain 17 recombinant virus treatment group; and the relative tumor inhibition rate was 39% in the HSV-mock group.

### 4. Oncolysis effects of oncolytic viruses expressing exogenous gene of anti-PD-1 monoclonal antibody

The ICP34.5 gene and ICP47 gene were knocked out from the virus strain with a CCTCC deposit number of V201810 (CCTCC: 201810) of the present application and the control virus strain 17 according to the method of Example 3, and a synthetic chemically synthesized exogenous gene was inserted at the position of the knocked-out ICP34.5 gene, and the anti-PD-1 monoclonal antibody exogenous gene include from 5' end to 3' end: CMV promoter, a gene encoding an anti-PD-1 antibody (J43, BioXCell), and BGHPolyA sequence. A homoplastic melanoma model was established by subcutaneous inoculation of B16F10 tumor cells in C57BL/6J mice. The control group was phosphate solution, and the treatment groups were anti-PD-1 monoclonal antibody (5 mg/kg), tested oncolytic virus HL1-mock (10⁷ pfu), HL1-PD1 (10⁷ pfu), HL1-PD1 (10⁶ pfu) and HL1-PD1 (10⁵ pfu), respectively, 10 mice in each group; the control and the tested oncolytic virus administration groups were administered by intratumoral injection. The anti-PD-1 monoclonal antibody administration group was administered intraperitoneally, and the relative tumor inhibition rates were calculated as described above.

Research Results: By analyzing the mean tumor volume of mice on day 13 after administration, there were statistically significant differences (P<0.05) in anti-tumor effects of the groups administered with each dose of HL1- PD1 (oncolytic viruses were administered alone) compared to that of the control group; groups with the high, medium and low doses of HL1-PD1 showed a significant dose effect with tumor inhibition rates of 86%, 65% and 51%, respectively, and the HL1-mock group and anti-PD-1 monoclonal antibody group had tumor inhibition rates of 34% and 42%, respectively.

### 5. Oncolysis effects of oncolytic viruses expressing exogenous genes of IL-12 and anti-PD-1 monoclonal antibodies

The ICP34.5 gene and ICP47 gene were knocked out from the virus strain with a CCTCC deposit number of V201810 (CCTCC: 201810) of the present application and the control virus strain 17 according to the method of Example 3, and the IL-12 gene and anti-PD-1 monoclonal antibody gene were inserted at the positions of ICP34.5 and ICP47 genes, respectively. The IC50 of HL1-IL12+PD1 (HL-1 inserted with IL-12 gene and anti-PD-1 monoclonal antibody gene), and 17-IL12+PD1 (strain 17 inserted with IL-12 gene and anti-PD-1 monoclonal antibody gene) transfecting different cells respectively were determined. The experimental cells included A-375 human skin cancer cells, A549 human lung cancer cells, AGS human gastric cancer cells, AsPC-1 human pancreatic cancer cells, HeLa human cervical cancer cells, and Hep G2 human liver cancer cells, HT-29 human colorectal cancer cells, MCF7 human breast cancer cells, PC-3 human prostate cancer cells, T24 human bladder cancer cells, U-2 OS human bone cancer cells and U-87 human glioma cells.

Cells in logarithmic phase were digested and passaged into 96-well plates. 24 hours later, the old medium was discarded and different viruses at 9 dilution gradients were added, 100 µl/well, making MOI 20, 5, 1.25, 0.3125, 0.0781, 0.0195, 0.0049, 0.0012 and 0.0003, and a blank control was set up. After culturing at 37°C for 3 days, all the liquid in the wells was discarded, 10% CCK-8 reagent was added, 100 µl/well, and they were incubated at 37°C for 1-2 h. The OD values of the samples were determined at 450 nm, and the IC50 value was calculated according to the fitted curve.

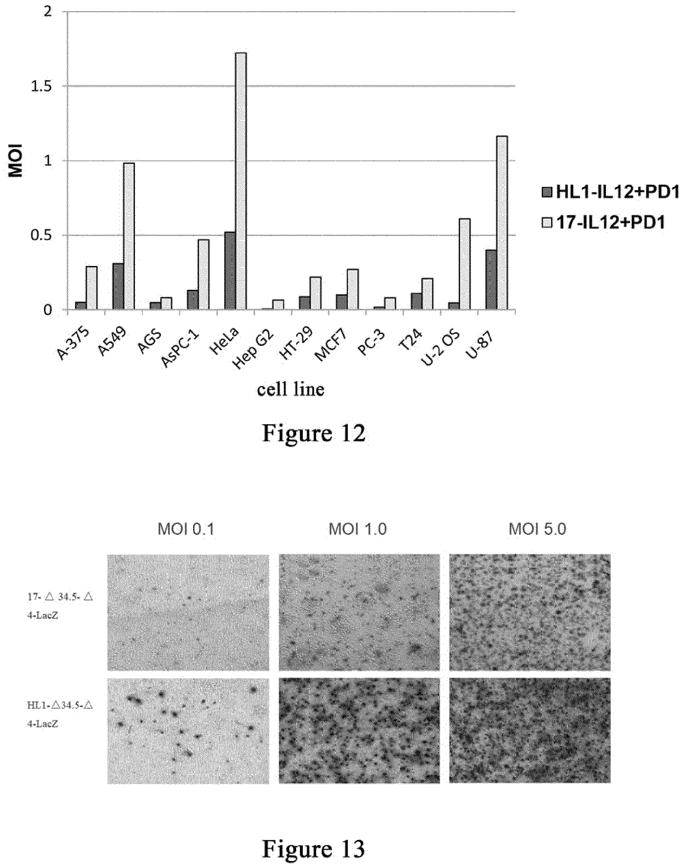

The purpose of this example is to compare the difference between the abilities of the virus strain HL-1 recombinant virus and the virus strain 17 recombinant virus to transfect tumor cells after insertion of IL-12 gene and anti-PD-1 monoclonal antibody gene. The IC50 values of the viruses transfecting tumor cells were determined by the CCK-8 method, and the results of the IC50 measurement results of different viruses transfecting tumor cells are shown in Figure 12. As shown in the results, the HL-1 recombinant virus inserted with the IL-12 gene and anti-PD-1 monoclonal antibody gene had lower IC50 value than that of the strain 17 recombinant virus inserted with the IL-12 gene and anti-PD-1 monoclonal antibody gene when transfecting tumor cells. It indicated that the strain HL-1 recombinant virus inserted with IL-12 gene and anti-PD-1 monoclonal antibody gene had better infection efficiency and replication ability in tumor cells, and had a strong oncolytic effect.

### Example 5 Comparison of the abilities of non-replicating HSV (HL-1) virus and HSV (17) virus to infect nerve cells

The purpose of this Example is to compare the abilities of the non-replicating HL-1 virus and strain 17 with different immediate early genes knockout to infect nerve cells, in order to compare the efficacy of the non-replicating HL-1 herpesvirus of the present application as a gene vector with the standard laboratory virus strain 17 of the prior art.

The non-replicating viruses were constructed according to methods 1 and 2, respectively, as follows:
1. The ICP34.5 and ICP4 genes of strain HL-1 and strain 17 HSV viruses were knocked out according to the method of Application Publication No. CN105219739A, and an exogenous reporter gene was inserted into the LAT region of the viruses. The gene includes from the 5' end to the 3' end: CMV promoter, gene encoding LacZ and BGH PolyA.
2. The ICP34.5 and ICP4 genes of strain HL-1 and strain 17 HSV viruses were knocked out according to the method of Application Publication No. CN105219739A, and an exogenous reporter gene was inserted into the LAT region of the viruses, and the ICP27 gene was knocked out at the same time (Howard M K, Kershaw T, Gibb B J, et al. High efficiency gene transfer to the central nervous system of rodents and primates using herpes virus vectors lacking functional ICP27 and ICP34.5.[J]. (Gene Therapy, 1998, 5(8): 1137-1147.). The gene include from the 5' end to the 3' end: the CMV promoter, the gene encoding LacZ, and BGH PolyA.

The abilities of the above recombinant viruses to transfect and express carried exogenous genes in primary nerve cells were compared. E14 rat cortex was taken, chopped and digested with trypsin, and after 15 minutes, complete medium was added and pipetted, and the cells were inoculated in culture dishes and replaced with nerve cell medium for culture. They were transfected with viruses at different MOI amounts, and the viruses were added and incubated in a 37°C, CO₂ incubator for 48 hours and then fixed and stained. They were taken out for microscopic observation, and were photographed and recorded.

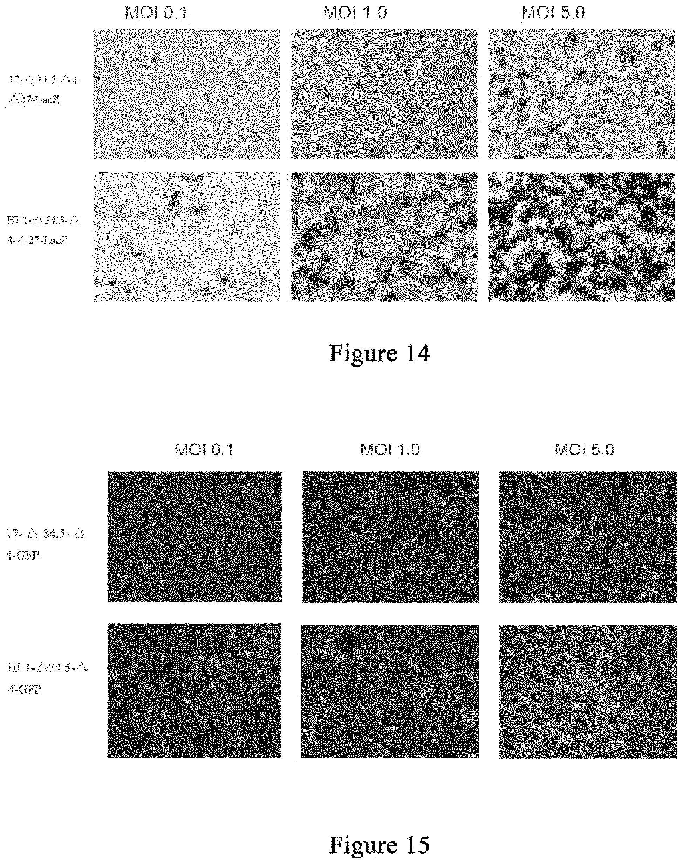

The results of the HL-1 recombinant virus and the strain 17 recombinant virus transfecting primary nerve cells are shown in Figure 13 and Figure 14. The experimental results showed that the HL-1 recombinant virus showed more blue spots in primary nerve cell cultures under different MOI transfection conditions, indicating that the HL-1 recombinant virus was more capable of transfecting nerve cells and also more capable of expressing carried exogenous genes compared to the strain 17 recombinant virus. The recombinant viruses constructed according to methods 1 and 2 above lost the ability to replicate in nerve cells, but could still transfect nerve cells at a high level and highly express the carried exogenous genes, and thus had good application prospects as gene therapeutic vectors for the treatment of nervous system diseases.

### Example 6 Comparison of the abilities of non-replicating HSV (HL-1) recombinant virus and HSV (17) recombinant virus to infect hepatocytes

The purpose of this Example is to compare the abilities of non-replicating HL-1 recombinant virus and strain 17 recombinant virus with different immediate early genes knockout to infect hepatocytes, in order to assess the potential of the non-replicating recombinant viruses of the present application as gene therapeutic vectors.

Non-replicating recombinant viruses were constructed according to methods 1 and 2 of Example 5, respectively, with the difference that the reporter gene was replaced with the GFP gene.

The abilities of the above recombinant viruses to transfect and express carried exogenous genes in CCC-HEL-1 (human fetal liver diploid cells) were compared. CCC-HEL-1 cells were inoculated in six-well plates, transfected with viruses at different MOI amounts, and incubated in a CO₂ incubator at 37°C for 48 h after adding viruses. The cells were taken out and observed under a fluorescence microscope, and photographed and recorded.

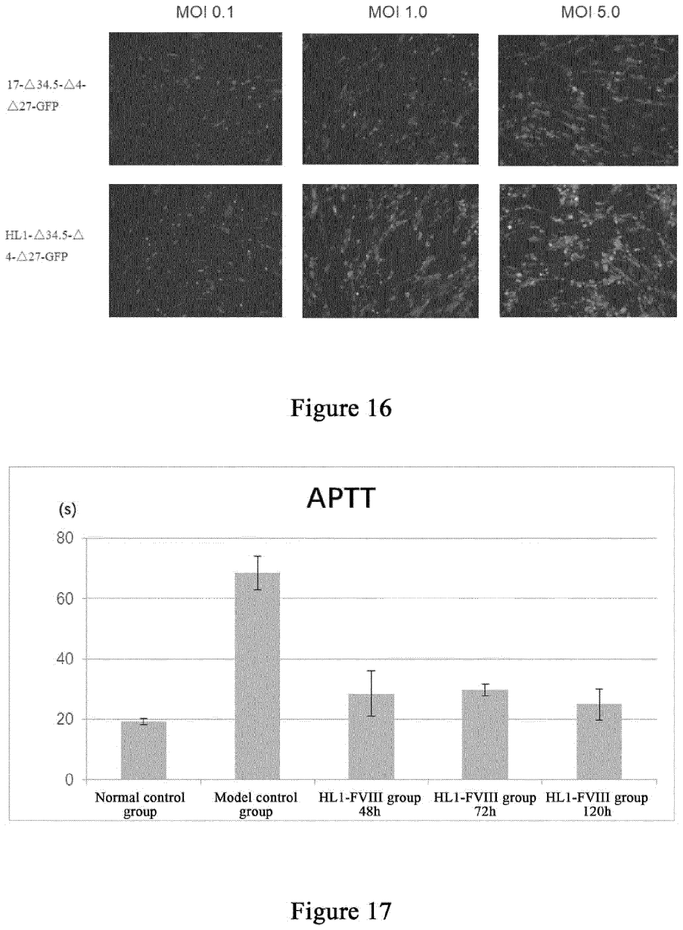

The results of the HL-1 recombinant virus and the strain 17 recombinant virus transfecting CCC-HEL-1 cells are shown in Figure 15 and Figure 16. The experimental results showed that under different MOI transfection conditions, the HL-1 recombinant virus showed more bright green fluorescence in CCC-HEL-1 cell cultures, indicating that the HL-1 recombinant virus was more capable of transfecting CCC-HEL-1 cells compared with the strain 17 recombinant virus. CCC-HEL-1 is a human fetal liver diploid normal cell, and the recombinant viruses constructed according to methods 1 and 2 above lost their ability to replicate in hepatocytes, but were still able to infect hepatocytes and express the exogenous genes they carried. The ability of the HL-1 recombinant virus to efficiently deliver therapeutic genes into target cells indicates that, as a vector, it can effectively deliver therapeutic genes into target cells to achieve the purpose of treating diseases.

### Example 7 Therapeutic effect of the virus strain HL-1 as a gene therapeutic vector carrying an exogenous gene

### 1. Procoagulant effect of HL1-FVIII on FVIII knockout mouse model

The ICP34.5 and ICP4 genes of the HL-1 virus were knocked out according to the method of Application Publication No. CN105219739A, and the FVIII gene was inserted in the latent expression region (LAT region) of the HL-1 virus. The aim of this Example is to observe the effect of HL1-FVIII on coagulation function in the FVIII knockout mouse model at different time points.

A total of 3 experimental groups, the normal control group, the model control group and the test sample group, were set up, with 5 experimental animals in each group. In the normal control group were C57BL/6 normal mice, and in the model control group and HL1-FVIII group were FVIII knockout model mice (Shanghai Model Organisms Center, Inc.). The administration was by intramuscular injection. The HL1-FVIII group was administered with the HL1-FVIII non-replicating HL-1 recombinant virus prepared as described above at 10⁷ pfu/each, while the other two groups were administered PBS. The administration was once every 3 days for 3 consecutive doses. The APTT was measured by blood collection after 48 h in the control group and 48 h, 72 h and 120 h in the test sample group.

The animals in each group were in good mental condition throughout the experiment. The activated partial thromboplastin time (APTT) reflects the sensitivity of the coagulation activity of the endogenous coagulation system, and the APTT values detected at different time periods are shown in Figure 17. The high APTT values in the model control group indicated an increased coagulation time due to the deficiency of factor F VIII *in vivo.* The experimental results showed that the APTT values in the group treated with the non-replicating HL-1 recombinant virus HL1-FVIII were substantially shorter compared to that of the un-treatment group and were close to the values in normal mice, indicating that the non-replicating HL-1 recombinant virus HL1-FVIII could consistently express factor FVIII in mice and had good clinical application prospect in the treatment of hemophilia A.

### 2. Analgesic effect of HL1-ENK in rats with chronic pain from arthritis

The ICP34.5 and ICP4 genes of the HL-1 virus were knocked out according to the method of Application Publication No. CN105219738A, and the enkephalin (ENK) gene was inserted into the latent expression region (LAT region) of the HL-1 virus. The aim of this Example is to observe the sustained analgesic effect of HL1-ENK in arthritic chronic pain rats.

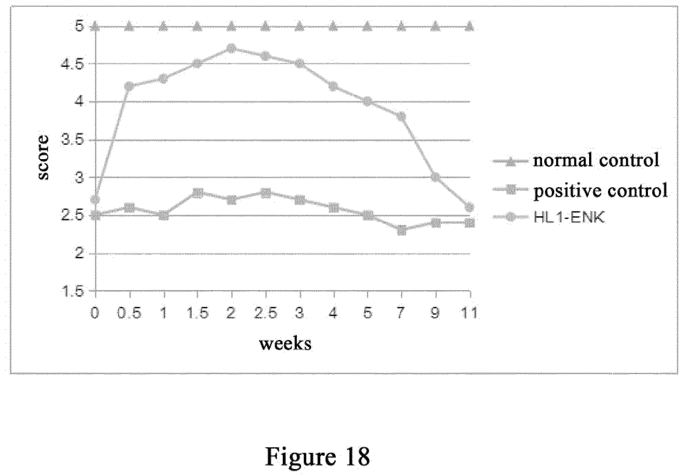

A total of three experimental groups, normal control group, model control group and test sample group, were set up. In the model control group and the test sample group, a fully Freund's adjuvant-induced arthritic chronic pain model rats were used, with six rats in each group. The administration was through the right hind foot pad, and 10⁷ pfu/each of the non-replicating recombinant virus HL1-ENK was administered to the test sample group, and PBS was administered to the other two groups as control. The rats were scored according to their behavioral ability status, with a score range of 1-5, with 5 being normal rat behavior and 1 being loss of normal mobility and severely impaired behavior. The higher the score, the more normal the activity is. Chronic pain rats suffering from arthritis will have their behavioral abilities affected due to pain. The behavior and activity scores of each experimental group are shown in Figure 18.

From the scoring of the rats' behavioral ability, the arthritic rats showed significant differences from the model control group 3 days after administration of the non-replicating recombinant virus HL1-ENK, with significant analgesic effects lasting more than 9 weeks, with peaks occurring at weeks 1-4, and behavioral scores not significantly different from those of normal rats.

Preferred embodiments of the present application are described in detail above, however, the present application is not limited to this. Within the technical conception of the present application, a variety of simple variants of the technical solutions of the present application, including the combination of individual technical features in any other suitable manner, may be made, and these simple variants and combinations shall likewise be considered as disclosed in the present application and all fall within the scope of protection of the present application.

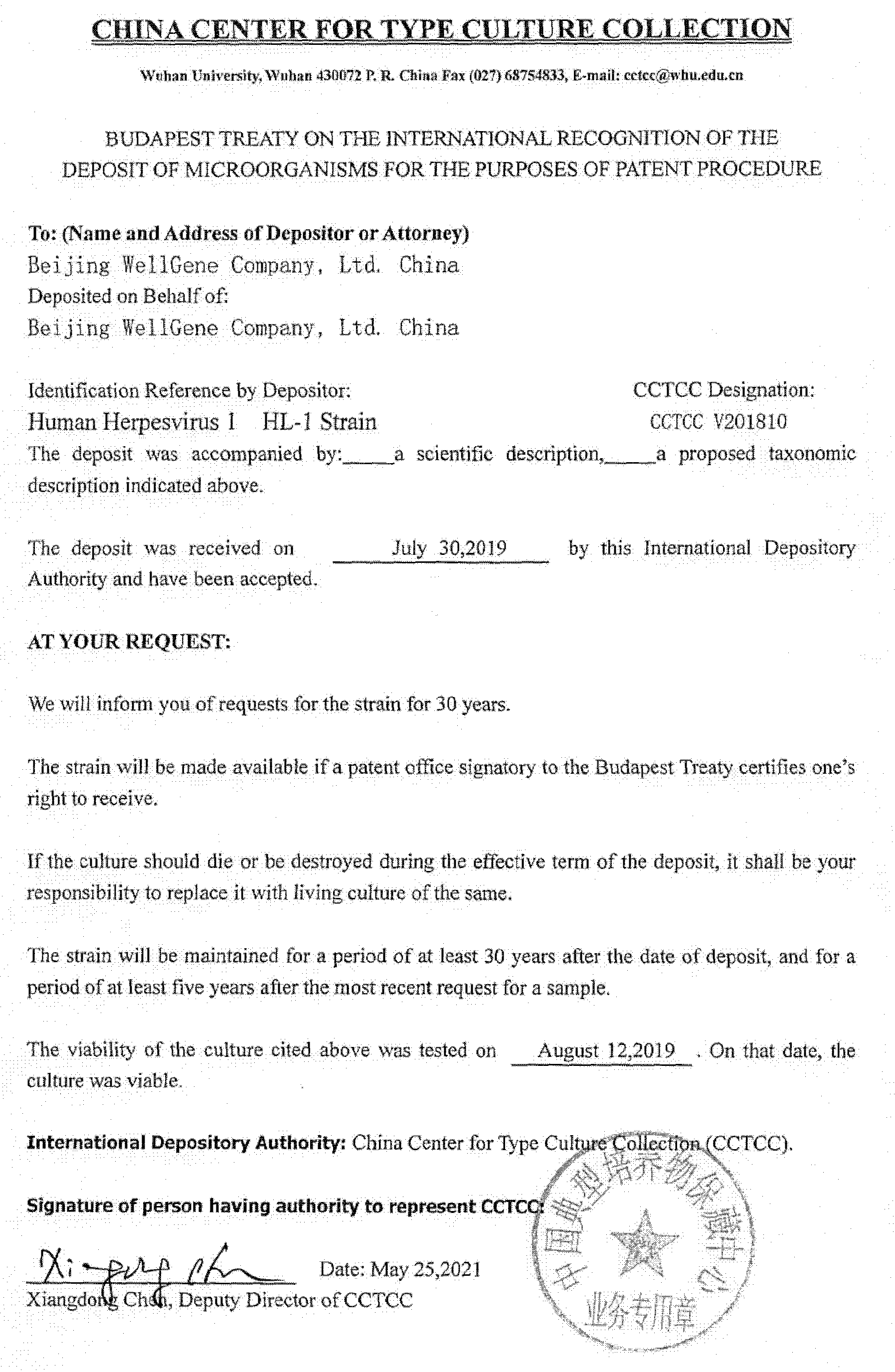
CHINA CENTER FOR TYPE CULTURE COLLECTION
Wuhan University, Wuhan 430072 P. R. China Fax (027) 68754833, E-mail: cctcc@whu.edu.cn

BUDAPEST TREATY ON THE INTERNATIONAL RECOGNITION OF THE
DEPOSIT OF MICROORGANISMS FOR THE PURPOSES OF PATENT PROCEDURE

To: (Name and Address of Depositor or Attorney)
Beijing WellGene Company, Ltd. China
Deposited on Behalf of:
Beijing WellGene Company, Ltd. China

Identification Reference by Depositor: | CCTCC Designation:
Human Herpesvirus 1 HL-1 Strain | CCTCC V201810
The deposit was accompanied by:_____a scientific description,_____a proposed taxonomic
description indicated above.

The deposit was received on July 30,2019 by this International Depository
Authority and have been accepted.

AT YOUR REQUEST:

We will inform you of requests for the strain for 30 years.

The strain will be made available if a patent office signatory to the Budapest Treaty certifies one's
right to receive.

If the culture should die or be destroyed during the effective term of the deposit, it shall be your
responsibility to replace it with living culture of the same.

The strain will be maintained for a period of at least 30 years after the date of deposit, and for a
period of at least five years after the most recent request for a sample.

The viability of the culture cited above was tested on August 12,2019 . On that date, the
culture was viable.

International Depository Authority: China Center for Type Culture Collection (CCTCC).

Signature of person having authority to represent CCTCC:

 Date: May 25,2021
Xiangdong Chen, Deputy Director of CCTCC

## Claims

1. A herpes simplex virus type 1 strain, which is a virus strain with a CCTCC deposit number of V201810 (CCTCC: V201810), or an attenuated strain thereof, or a culture progeny thereof.

2. A genetically modified herpes simplex virus, wherein one or more genes selected from the group consisting of: ICP34.5 gene, ICP6 gene, ICP0 gene, ICP47 gene, US3 gene, UL56 gene, functional VP16 gene, VHS gene, UNG gene, glycoprotein H gene, and thymidine kinase gene are deleted from the herpes simplex virus, wherein the genetically modified herpes simplex herpesvirus is derived from a herpes simplex virus with a CCTCC deposit number of V201810 or an attenuated strain thereof, or a culture progeny thereof.

3. The genetically modified herpes simplex virus of claim 2, wherein the modification is deletion of the ICP34.5 gene and/or the ICP47 gene in the herpes simplex virus.

4. The genetically modified herpes simplex virus of claim 2 or 3, wherein the modification further comprises introducing one or more exogenous genes into the virus.

5. The genetically modified herpes simplex virus of claim 4, wherein the one or more exogenous genes are inserted into one or more deletion sites of a gene selected from the group consisting of: ICP34.5 gene, ICP6 gene, ICP0 gene, ICP47 gene, US3 gene, UL56 gene, VP16 gene, VHS gene, UNG gene, glycoprotein H gene, and thymidine kinase gene.

6. The genetically modified herpes simplex virus of any one of claims 2-5, wherein the exogenous genes are one or more genes selected from the group consisting of: genes encoding cytokines promoting an immune response, genes encoding tumor antigens, genes encoding monoclonal antibodies having prophylactic and/or therapeutic effects against tumors, genes encoding prodrug-converting enzymes, tumor suppressor genes, antisense RNAs, and small RNAs.

7. The genetically modified herpes simplex virus of claim 6, wherein the cytokines are one or more cytokines selected from the group consisting of: GM-CSF, G-CSF, M-CSF, IL-1, IL-2, IL-3, IL-4, IL-5, IL-6, IL7, IL-8, IL-10, IL-12, IL-13, IL-15, IL-18, IL- 21, IL-23, IFN-α, IFN-γ, TGF-β, and TNF-α.

8. The genetically modified herpes simplex virus of claim 6, wherein the monoclonal antibodies having prophylactic and/or therapeutic effects against tumors are one or more antibodies selected from the group consisting of: anti-PD-1 monoclonal antibodies, anti-PD-L1 monoclonal antibodies, anti-PD-L2 monoclonal antibodies, anti-CTLA-4 monoclonal antibodies, anti-CD80 monoclonal antibodies, anti-CD28 monoclonal antibodies, anti-CD 137 monoclonal antibodies, anti-CD137L monoclonal antibodies, anti-OX40 monoclonal antibodies, anti-OX40L monoclonal antibodies, anti-CD27 monoclonal antibodies, anti-CD70 monoclonal antibodies, anti-CD40 monoclonal antibodies, anti-CD40L monoclonal antibodies, anti-LAG-3 monoclonal antibodies, and anti-TIM-3 monoclonal antibodies.

9. The genetically modified herpes simplex virus of claim 6, wherein the tumor antigens are tumor-specific antigens.

10. The genetically modified herpes simplex virus of claim 9, wherein the tumor-specific antigens are one or more antigens selected from the group consisting of: PSA, MUC1, MAGE-1, MAGE-2, MAGE-3, MAGE-12, BAGE, GAGE, and LAGE.

11. The genetically modified herpes simplex virus of any one of claims 4-10, wherein the exogenous genes encode a polypeptide selected from the group consisting of: cytokine polypeptides stimulating immune response and antibody polypeptides promoting immune response.

12. The genetically modified herpes simplex virus of claim 11, wherein the exogenous genes are one or more exogenous genes selected from the group consisting of: granulocyte macrophage colony stimulating factor (GMCSF), IL-2, IL-12, IFN-γ, TNF-α, and immune checkpoint antibody polypeptides.

13. The genetically modified herpes simplex virus of any one of claims 2-5, wherein one or more immediate early genes are knocked out or disrupted thereby losing the ability to replicate.

14. The genetically modified herpes simplex virus of claim 13, wherein the one or more immediate early genes are one, two, three or four immediate early genes selected from the group consisting of genes encoding ICP0, ICP4, ICP22 and ICP27.

15. The genetically modified herpes simplex virus of claim 13 or 14, wherein the exogenous genes are one or more exogenous genes selected from the group consisting of: nerve growth factor genes, neurotransmitter genes, and neurotrophic factor genes.

16. The genetically modified herpes simplex virus of claim 13 or 14, wherein the exogenous genes are coagulation factor genes.

17. A non-replicating herpes simplex virus for use as a vector, wherein one, two, or three genes selected from the group consisting of: ICP4, ICP27, and ICP34.5 are knocked out or disrupted, and the non-replicating herpes simplex virus is derived from a herpes simplex virus with a CCTCC deposit number of V201810 or an attenuated strain thereof, or a progeny thereof.

18. The herpes simplex virus of claim 17, wherein one or more exogenous genes are further introduced.

19. The herpes simplex virus of claim 18, wherein the exogenous genes are one or more exogenous genes selected from the group consisting of: nerve growth factor genes, neurotransmitter genes, neurotrophic factor genes, and coagulation factor genes.

20. A composition, virus culture, host cell, or host cell culture comprising the herpes simplex virus of any one of claims 1-19.

21. A method for treating a cancer, comprising introducing the herpes simplex virus of any one of claims 1-12 into a subject suffering from a cancer.

22. A method for treating a nervous system disease, a hematological system disease, or an immune system disease, comprising introducing the herpes simplex virus of any one of claims 15-19 into a subject.

23. The method of claim 22, wherein the hematological system disease is a coagulation disorder.

24. The method of claim 22, wherein the nervous system disease is chronic pain, nerve injury, stroke, paralysis or a neurodegenerative disease.

25. The method of claim 24, wherein the neurodegenerative disease is Parkinson's disease, Alzheimer's disease, amyotrophic lateral sclerosis, or Huntington's disease.
